# EUROPEAN PATENT APPLICATION

(11) **EP 4 628 492 A1**
(43) Date of publication of application: **08.10.2025**
(21) Application number: 23910850.9
(22) Date of filing: 28.12.2023
(51) Int. Cl.: C07D 491/22, C07C 315/04, C07C 317/28, C07K 5/062, C07K 1/107, C07K 1/16, C07K 5/033

(54) **PREPARATION METHOD FOR LINKER-DRUG CONJUGATE AND INTERMEDIATE THEREOF**

(30) Priority: 30.12.2022 CN 202211728484
(71) Applicant: Shanghai Fudan-Zhangjiang Bio-Pharmaceutical Co., Ltd., Shanghai 201210 (CN)
(72) Inventor: LIU, Ning, Shanghai 201210 (CN); LI, Zhendong, Shanghai 201210 (CN); SHEN, Lei, Shanghai 201210 (CN); YANG, Zhou, Shanghai 201210 (CN); WANG, Dongsheng, Shanghai 201210 (CN); QIU, Xuefei, Shanghai 201210 (CN); WANG, Baoxia, Shanghai 201210 (CN)
(74) Representative: Dehns
(86) International application number: PCT/CN2023/142851
(87) International publication number: WO 2024/140934

(57) **Abstract**

Provided are a preparation method for a linker-drug conjugate and an intermediate thereof. Particularly provided is a preparation method for a compound LE14, comprising subjecting an intermediate, i.e., a compound of formula II, and a compound of formula III or mesylate thereof to an amide condensation reaction in the presence of a condensation agent, an alkali and a solvent so as to obtain a compound of formula I. The preparation method can prepare the high-purity intermediate II, then directly condense the intermediate II with exatecan mesylate to obtain the compound I, and then continue to perform subsequent reactions, so as to prepare the final product LE14. The preparation method has one or more following advantages: using the intermediate II as an initial raw material of process production reduces process steps, facilitates control of product quality, and remarkably reduces impurities in the final product so as to improve the purity of the final product; and using the more economical exatecan for condensation greatly lowers the production cost and makes the method more applicable to industrial production.

## Description

The present application claims the right of the priority of the Chinese patent application 2022117284842 filed on December 30, 2022. The contents of the Chinese patent application are incorporated herein by reference in its entirety.

### TECHNICAL FIELD

The present disclosure belongs to the field of drug synthesis, and specifically relates to a preparation method for a linker-drug conjugate and an intermediate thereof.

### BACKGROUND

Antibody-drug conjugates (ADCs) have emerged as a highly competitive drug pipeline that domestic and foreign pharmaceutical companies are racing to develop in recent years, becoming one of the hot spots in the pharmaceutical industry. ADC drugs consist of three fundamental modules: an antibody, a linker, and an effector molecule. ADC drugs use the specific targeting effect of antibodies to transport the effector molecule to the tumor site for enrichment, thereby achieving the purpose of killing tumor cells. Among effector molecules, a class of camptothecin analogs are widely used in the ADC field. In recent years, Immunomedics has used it as an effector molecule in its ADC drug IMMU-132 (ZL200980156218), showing good antitumor effects. Daiichi Sankyo has used another camptothecin analog as the effector molecule in its ADC drug DS-8201a (ZL201380053256), also showing good antitumor effects.

Patent application WO2020259258A1 discloses an ADC compound using camptothecin derivative DXd as the effector molecule and provides the preparation methods for the linker-drug conjugate LE14 as shown in Route 1 and Route 2. Patent applications WO2022204947A1, CN115215921A, and CN115385926A provide improved Routes 3, 4, and 5.

### Route 1:

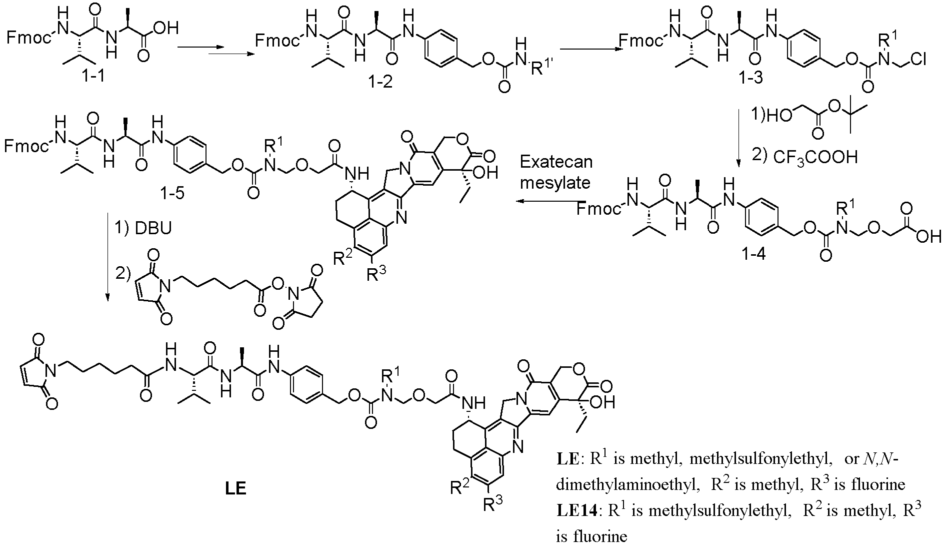

The synthesis method of Route 1 comprises the following steps: reacting compound **1-1** with 4-aminobenzyl alcohol, further reacting the resulting compound with bis(p-nitrophenyl) carbonate, then reacting with a substituted alkylamine to obtain compound **1-2,** reacting compound **1-2** with paraformaldehyde and trimethylsilyl chloride to obtain compound **1-3,** reacting compound **1-3** with *tert*-butyl glycolate, subsequently removing the *tert*-butyl group under the action of trifluoroacetic acid to obtain compound **1-4,** reacting compound **1-4** with exatecan mesylate to obtain compound **1-5,** removing the Fmoc protecting group on the amino group under the action of DBU, and finally performing a coupling reaction with *N-*succinimidyl 6-maleimidohexanoate to obtain the target compound **LE14.**

In the step of using intermediate **1-3** as the raw material to prepare intermediate **1-4** in this route, since the raw material and the product are unstable to both acids and bases, there is a certain risk of decomposition during deprotection and purification processes. Compound **1-4** is not purified, and the crude product is directly used in the next reaction, leading to the formation of significant impurities in the crude product of compound **1-5.** These impurities have polarities close to the product, making purification difficult and consequently affecting the product quality **of LE14.**

### Route 2:

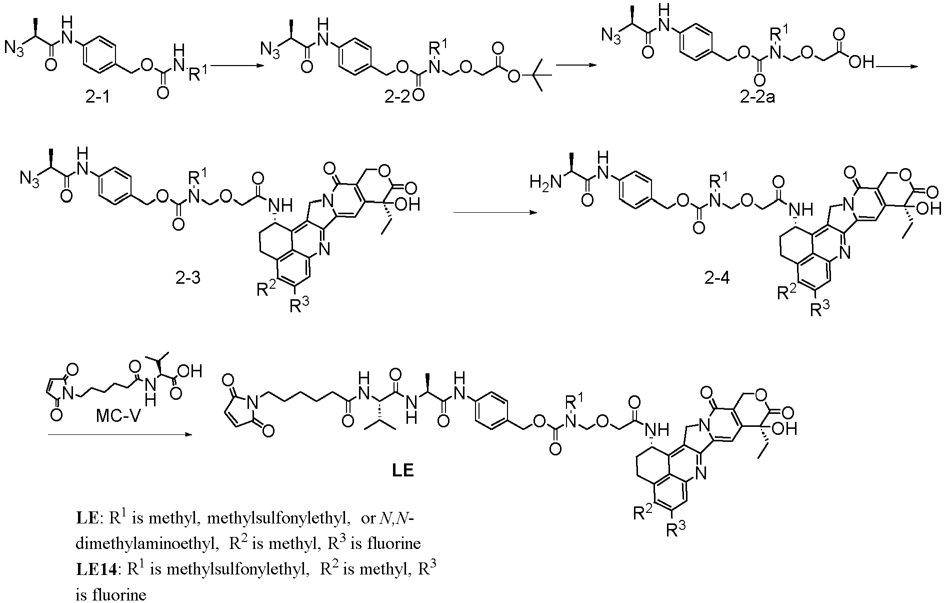

The synthesis method of Route 2 comprises the following steps: reacting compound 2-1 with paraformaldehyde and trimethylsilyl chloride, further reacting the resulting compound with *tert*-butyl glycolate to obtain compound **2-2,** removing the *tert-butyl* group from compound **2-2** under the action of trifluoroacetic acid to obtain compound **2-2a,** then reacting compound **2-2a** with exatecan mesylate to obtain compound **2-3,** reducing the azide group to the amino group in compound **2-3** under the action of triethylphosphine to obtain compound **2-4,** and finally performing a coupling reaction between compound **2-4** and MC-V to obtain the target compound **LE14.**

In this route, the raw material **2-2** and the carboxylic acid intermediate **2-2a** generated by the removal of the *tert*-butyl group are unstable to both acids and bases. There is a certain risk of decomposition during deprotection and purification processes. Compound **2-2a** is not purified, and the crude product is directly used in the next reaction, leading to the formation of significant impurities in the crude product of compound **2-3.** These impurities have polarities close to the product, making purification difficult and consequently affecting the product quality of**LE14**.

### Route 3:

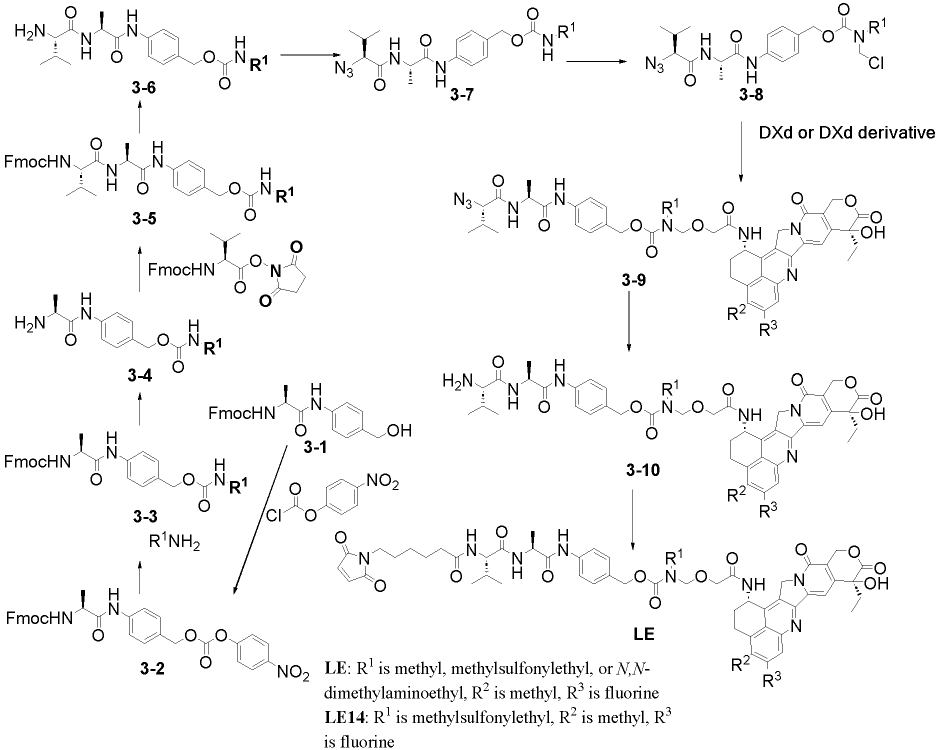

The synthetic method of Route 3 comprises the following steps: reacting compound **3-1** with *p*-nitrophenyl chloroformate to obtain compound **3-2,** reacting the resulting compound with the corresponding amine to obtain compound **3-3,** deprotecting compound **3-3** to obtain compound **3-4,** further reacting compound **3-4** with an amino acid active ester to obtain compound **3-5,** deprotecting compound **3-5** to obtain compound **3-6,** subsequently reacting compound **3-6** with an acyl azide reagent to obtain compound **3-7,** performing a chloromethylation reaction to obtain compound **3-8,** reacting compound **3-8** with DXd or a DXd derivative to obtain compound **3-9,** reducing the azide group in compound **3-9** to obtain compound **3-10,** and finally attaching the maleimide linker to obtain the final product **LE14.**

Following the method of Route 3, the overall yield **of LE14** prepared from compound **3-1** as the starting material is 3.5%, while the overall yield **of LE14** prepared from compound **3-7** as the starting material is 10.7%.

In this route, DXd or a DXd derivative is used as the source of payload, which is expensive and greatly increases the cost of preparing **LE14.**

### Route 4:

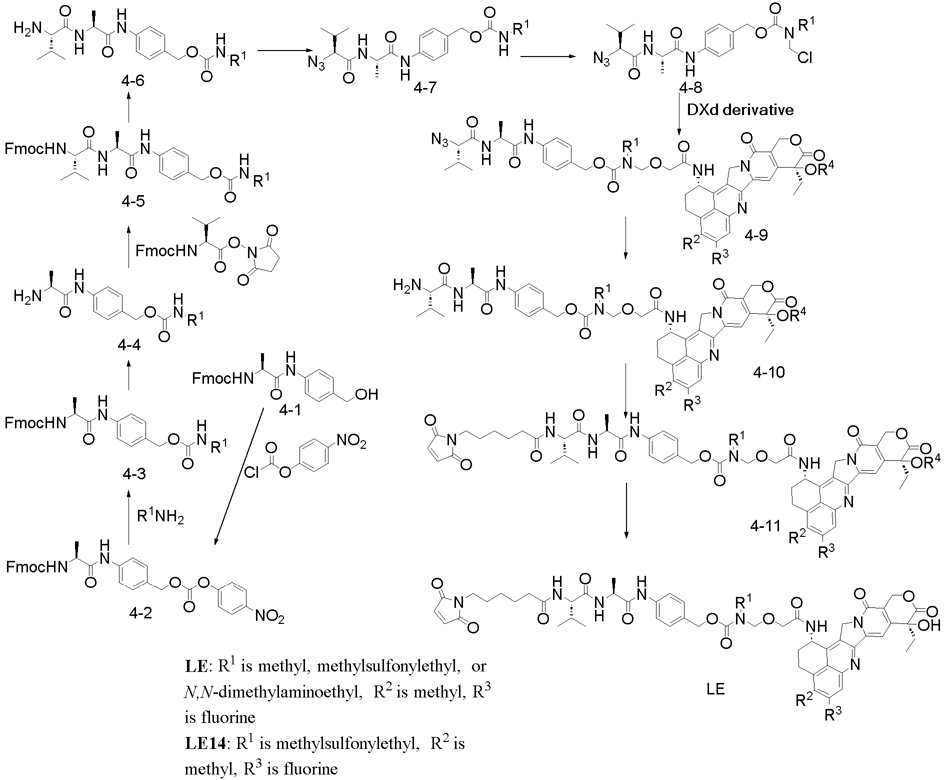

The synthetic method of Route 4 comprises the following steps: reacting compound 4-1 withp-nitrophenyl chloroformate to obtain compound **4-2,** reacting the resulting compound with the corresponding amine to obtain compound **4-3,** deprotecting compound **4-3** to obtain compound **4-4,** further reacting compound **4-4** with an amino acid active ester to obtain compound **4-5,** deprotecting compound **4-5** to obtain compound **4-6,** subsequently reacting compound **4-6** with an acyl azide reagent to obtain compound **4-7,** performing a chloromethylation reaction to obtain compound **4-8,** reacting compound **4-8** with a DXd derivative to obtain compound **4-9,** reducing the azide group in compound **4-9** to obtain compound **4-10,** then attaching the maleimide linker to obtain compound **4-11,** and finally removing the hydroxyl protecting group to obtain the final product **LE14.**

Following the method of Route 4, using compound **4-1** as the starting material and Dxd-a as the payload source, the overall yield for preparing **LE14** is 7.3% (**4-1→Dxd-a→LE14**). Using compound **4-1** as the starting material and Dxd-b as the payload source, the overall yield for preparing **LE14** is 12.9% (**4-1→Dxd-b→LE14**).

In this route, DXd derivative Dxd-a (structure shown in Route 4-a) or Dxd-b (structure shown in Route 4-b) is used as the payload source, which is expensive and greatly increases the cost of preparing **LE14.**

To avoid purchasing expensive DXd derivatives, our company has developed synthetic routes 4-a and 4-b for the preparation of Dxd-a and Dxd-b starting from exatecan.

### Derivatization Route 4-a of Exatecan: (Exatecan→Dxd-a)

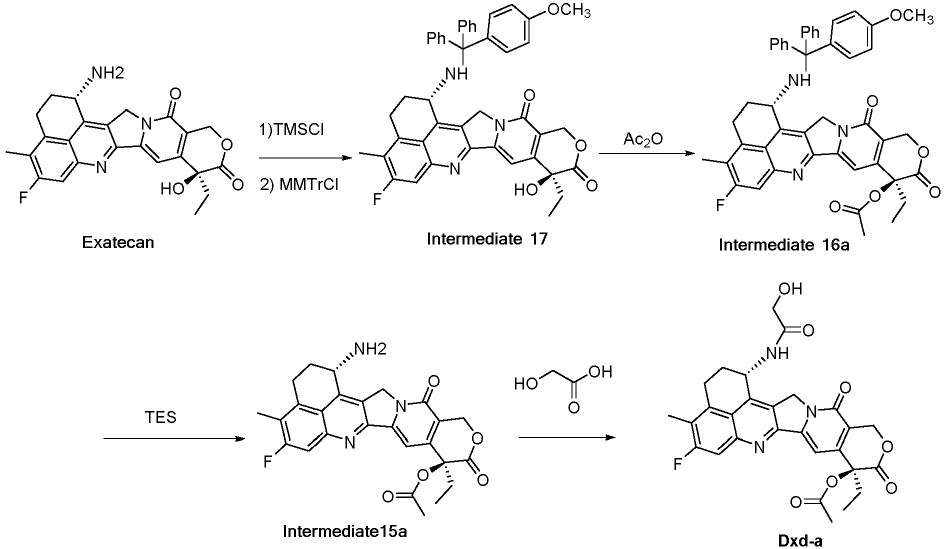

The synthetic method for preparing Dxd-a according to Route 4-a comprises: reacting exatecan or its mesylate with 4-methoxytriphenylmethyl chloride in the presence of trimethylsilyl chloride and *N,N-diisopropylethylamine* to obtain the amino-protected intermediate compound 17, reacting compound 17 with acetic anhydride under alkaline conditions to obtain the acetylated intermediate 16a, removing the amino protecting group from intermediate **16a** under the action of triethylsilane to obtain intermediate **15a,** further reacting intermediate **15a** with glycolic acid to obtain compound Dxd-a. The overall yield of Dxd-a prepared from exatecan as the starting material is 38.3% (exatecan→Dxd-a; hereafter referred to as the yield of Route 4-a).

The **LE14** is prepared according to the method of Route 4, using compound **4-1** as the starting material to obtain compound **4-8** (according to the synthetic method of Route 4) and using Dxd-a as the payload source prepared from exatecan as the starting material (according to the synthesis method of Route 4-a). The overall yield for preparing **LE14** is 2.8% (the yield of **4-1→Dxd-a→LE14** multiplied by the yield of the derivatization Route 4-a of exatecan, *i.e.,* 7.3% × 38.3% = 2.8%).

Using compound **4-7** as the starting material and exatecan as the starting material according to the synthetic method of Route 4-a, the overall yield for preparing **LE14** with Dxd-a as the payload source according to the method of Route 4 is 8.2% (the yield of **4-7→Dxd-a→LE14** multiplied by the yield of the derivatization Route 4-a of exatecan, *i.e.,* 21.3% × 38.3% = 8.2%).

### Derivatization Route 4-b of Exatecan: (Exatecan→Dxd-b)

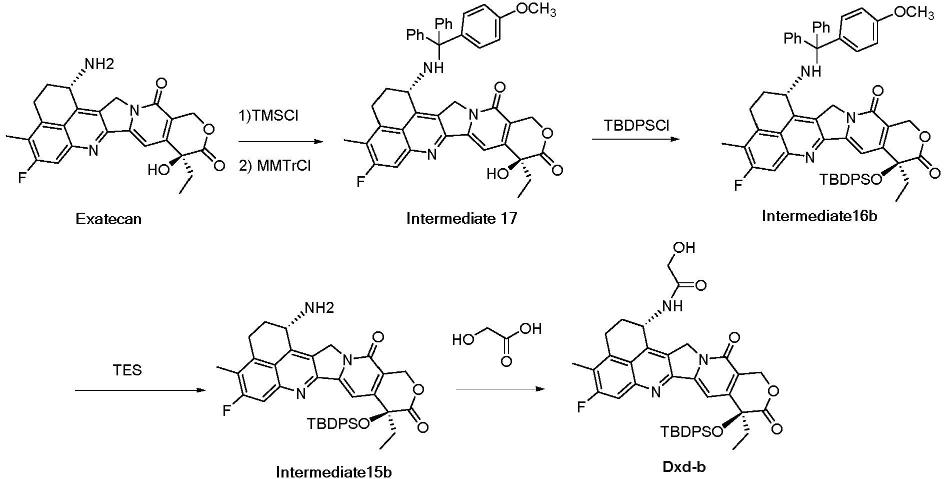

The synthetic method for preparing Dxd-b according to Route 4-b comprises: reacting exatecan or its mesylate with 4-methoxytriphenylmethyl chloride in the presence of trimethylsilyl chloride and *N,N-diisopropylethylamine* to obtain the amino-protected intermediate compound 17, reacting compound **17** with tert-butyldiphenylsilyl chloride under alkaline conditions to obtain the *tert*-butyldiphenylsilylated intermediate **16b,** removing the amino protecting group from intermediate **16b** under the action of triethylsilane to obtain intermediate **15b,** further reacting intermediate **15b** with glycolic acid to obtain compound Dxd-b. The overall yield of Dxd-b prepared from exatecan as the starting material is 48.2% (exatecan→Dxd-b; hereafter referred to as the yield of Route 4-b).

The **LE14** is prepared according to the method of Route 4, using compound **4-1** as the starting material to obtain compound **4-8** (according to the synthetic method of Route 4) and using Dxd-b as the payload source prepared from exatecan as the starting material (according to the synthesis method of Route 4-b). The overall yield for preparing **LE14** is 6.2% (the yield of **4-1→Dxd-b→LE14** multiplied by the yield of the derivatization Route 4-b of exatecan, *i.e.,* 12.9% × 48.2% = 6.2%).

Using compound **4-7** as the starting material and exatecan as the starting material according to the synthetic method of Route 4-b, the overall yield for preparing **LE14** with Dxd-b as the payload source according to the method of Route 4 is 18.0% (the yield of **4-7→Dxd-b→LE14** multiplied by the yield of the derivatization Route 4-b of exatecan, *i.e.,* 37.5% × 48.2% = 18.0%).

If using exatecan as the starting material to prepare Dxd-a and Dxd-b according to the methods of Routes 4-a and 4-b as the source of Dxd derivatives for Route 4, although it avoids the direct use of the more expensive Dxd, it adds four additional reaction steps, increases the experimental workload, and also results in significant losses during the derivatization process of exatecan, thereby raising production costs instead.

### Route 5:

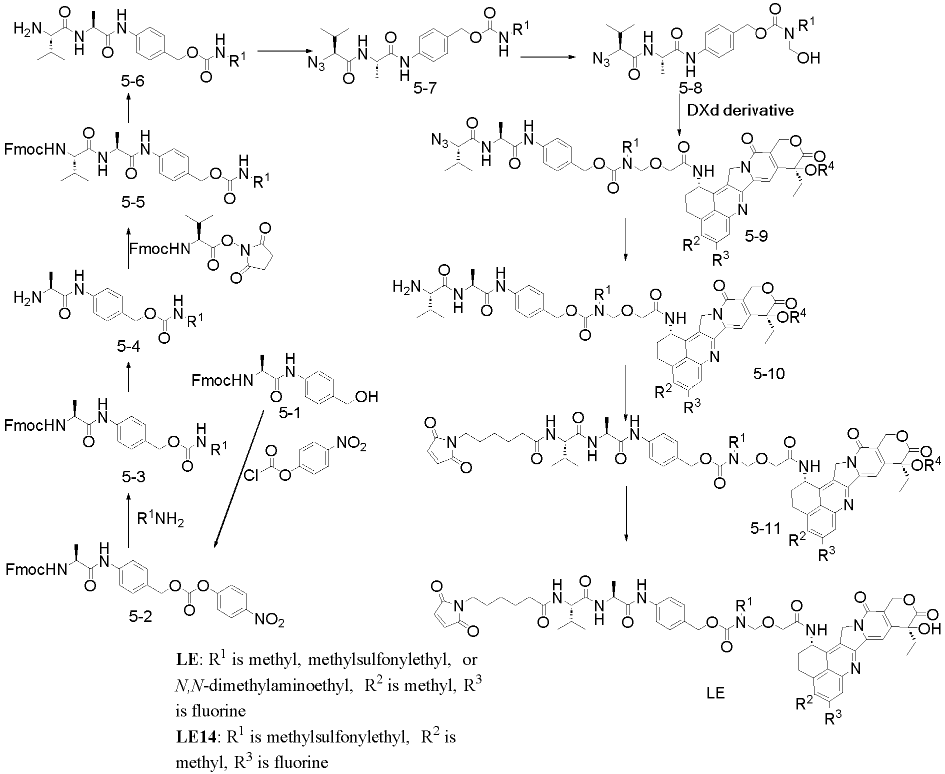

The synthetic method of Route 5 comprises the following steps: reacting compound **5-1** with *p*-nitrophenyl chloroformate to obtain compound **5-2,** reacting the resulting compound with the corresponding amine to obtain compound **5-3,** deprotecting compound **5-3** to obtain compound **5-4,** further reacting compound **5-4** with an amino acid active ester to obtain compound **5-5,** deprotecting compound **5-5** to obtain compound **5-6,** subsequently reacting compound **5-6** with an acyl azide reagent to obtain compound **5-7,** performing a hydroxymethylation reaction to obtain compound **5-8,** reacting compound **5-8** with a DXd derivative to obtain compound **5-9,** reducing the azide group in compound **5-9** to obtain compound **5-10,** then attaching the maleimide linker to obtain compound **5-11,** and finally removing the hydroxyl protecting group to obtain the final product **LE14.**

Following the method of Route 5, using compound 5-1 as the starting material, the overall yield for preparing **LE14** with Dxd-a' (numbered as Dxd-a in the original patent application, structure shown in Route 5-a, and renumbered as Dxd-a' in the present disclosure to distinguish it from Dxd-a in Route 4-a) as the payload source is 6.9%; using compound 5-1 as the starting material, the overall yield for preparing **LE14** with Dxd-b' (numbered as Dxd-b in the original patent application, structure shown in Route 5-b, and renumbered as Dxd-b' in the present disclosure to distinguish it from Dxd-b in Route 4-b) as the payload source is 12.2%.

In this route, DXd derivative Dxd-a' or Dxd-b' is used as the payload source, which is expensive and greatly increases the cost of preparing **LE14.**

To avoid purchasing expensive DXd derivatives, our company has developed synthetic routes 5-a and 5-b for the preparation of Dxd-a' and Dxd-b' starting from exatecan.

### Derivatization Route 5-a of Exatecan: (Exatecan→Dxd-a')

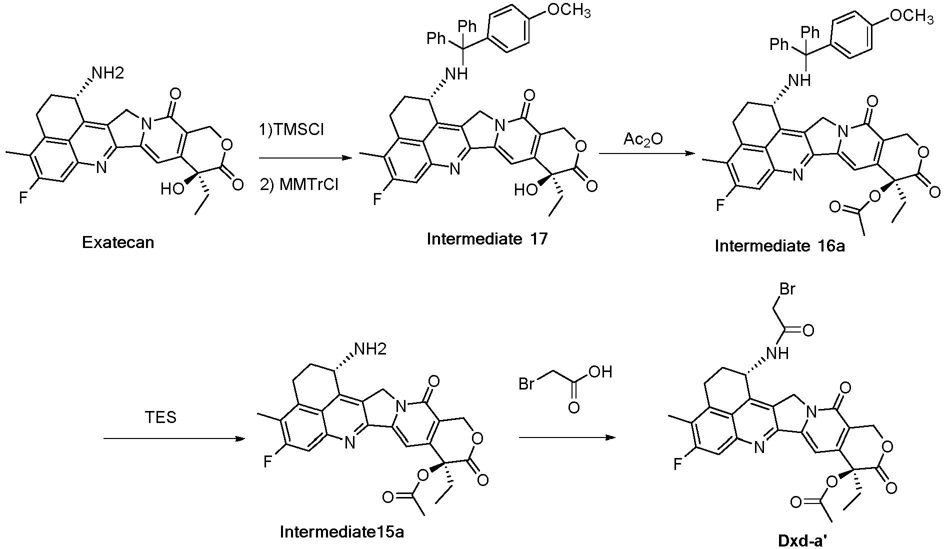

The synthetic method for preparing Dxd-a' according to Route 5-a comprises: reacting exatecan or its mesylate with 4-methoxytriphenylmethyl chloride in the presence of trimethylsilyl chloride and *N,N-diisopropylethylamine* to obtain the amino-protected intermediate compound **17,** reacting compound **17** with acetic anhydride under alkaline conditions to obtain the acetylated intermediate **16a,** removing the amino protecting group from intermediate **16a** under the action of triethylsilane to obtain intermediate **15a,** further reacting intermediate **15a** with 2-bromoacetic acid to obtain compound Dxd-a'. The overall yield of Dxd-a' prepared from exatecan as the starting material is 27.6% (**exatecan→Dxd-a',** hereafter referred to as the yield of Route 5-a).

The **LE14** is prepared according to the method of Route 5, using compound **5-1** as the starting material to obtain compound **5-8** (according to the synthetic method of Route 5) and using Dxd-a' as the payload source prepared from exatecan as the starting material (according to the synthesis method of Route 5-a). The overall yield for preparing **LE14** is 1.9% (the yield of **5-1→Dxd-a'→LE14** multiplied by the yield of the derivatization Route 5-a of exatecan, *i.e.*, 6.9% × 27.6% = 1.9%).

Using compound **5-7** as the starting material and exatecan as the starting material according to the synthetic method of Route 5-a, the overall yield for preparing **LE14** with Dxd-a' as the payload source according to the method of Route 5 is 6.5% (the yield of **5-7→Dxd-a'→LE14** multiplied by the yield of the derivatization Route 5-a of exatecan, *i.e.,* 23.4% × 27.6% = 6.5%).

### Derivatization Route 5-b of Exatecan: (Exatecan→Dxd-b')

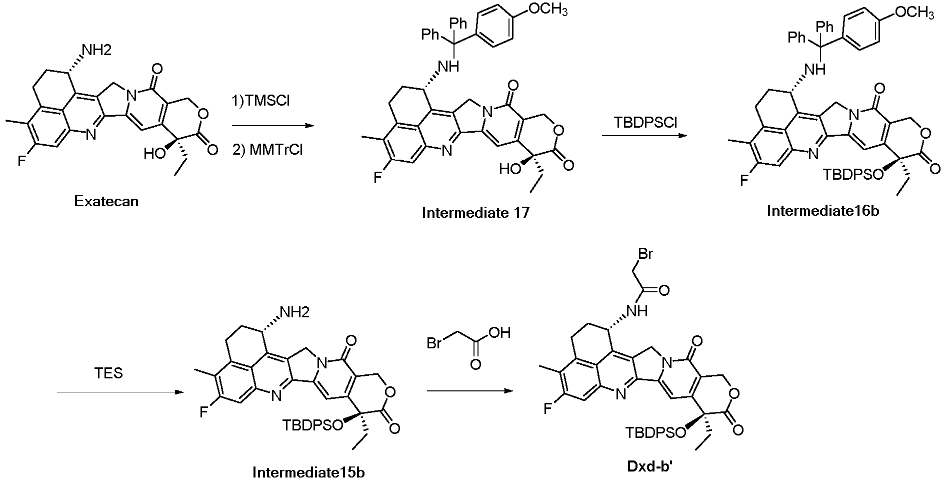

The synthetic method for preparing Dxd-b' according to Route 5-b comprises: reacting exatecan or its mesylate with 4-methoxytriphenylmethyl chloride in the presence of trimethylsilyl chloride and *N,N-diisopropylethylamine* to obtain the amino-protected intermediate compound 17, reacting compound 17 with tert-butyldiphenylsilyl chloride under alkaline conditions to obtain the *tert*-butyldiphenylsilylated intermediate **16b,** removing the amino protecting group from intermediate **16b** under the action of triethylsilane to obtain intermediate **15b,** further reacting intermediate **15b** with 2-bromoacetic acid to obtain compound Dxd-b'. The overall yield of Dxd-b' prepared from exatecan as the starting material is 44.5% (**exatecan→Dxd-b**', hereafter referred to as the yield of Route 5-b).

The **LE14** is prepared according to the method of Route 5, using compound **5-1** as the starting material to obtain compound **5-8** (according to the synthetic method of Route 5) and using Dxd-b' as the payload source prepared from exatecan as the starting material (according to the synthesis method of Route 5-b). The overall yield for preparing **LE14** is 5.4% (the yield of **5-1→Dxd-b'→LE14** multiplied by the yield of the derivatization Route 5-b of exatecan, *i.e.,* 12.2% × 44.5% = 5.4%).

Using compound **5-7** as the starting material and exatecan as the starting material according to the synthetic method of Route 5-b, the overall yield for preparing **LE14** with Dxd-b' as the payload source according to the method of Route 5 is 18.3% (the yield of **5-7→Dxd-b'→LE14** multiplied by the yield of the derivatization Route 5-b of exatecan, *i.e.,* 41.2% × 44.5% = 18.3%).

If using exatecan as the starting material to prepare Dxd-a' and Dxd-b' according to the methods of Routes 5-a and 5-2 as the source of Dxd derivatives for Route 5, although it avoids the direct use of the more expensive Dxd, it adds four additional reaction steps, increases the experimental workload, and also results in significant losses during the derivatization process of exatecan, thereby raising production costs instead.

After verification and evaluation, the following deficiencies have been found in the above routes:

In Route 1 and Route 2, intermediates 1-3 and 2-2 require the removal of the *tert*-butyl group to prepare the carboxylic acid intermediates. However, this reaction uses trifluoroacetic acid for deprotection, which involves harsh conditions, and the trifluoroacetic acid is difficult to remove by post-treatment. At the same time, the generated carboxylic acid intermediates 1-4 and 2-2a are unstable and nearly completely lost during purification, necessitating the use of unpurified crude products directly in the next reaction. This leads to the presence of impurities in the final product with polarities similar to the target compound, increasing the difficulty of purification. At the same time, Routes 3, 4, and 5 require the use of DXd or DXd derivatives, significantly increasing production costs.

To obtain a purified and stable carboxylic acid intermediate, we also attempted to protect the carboxyl group with methyl or ethyl groups. For example, we reacted the methanesulfonylethylamine intermediate with methyl glycolate or ethyl glycolate, followed by ester hydrolysis under basic or acidic conditions to obtain the carboxylic acid. However, both the ester structure and the resulting carboxylic acid after hydrolysis were unstable to both acids and bases, making it impossible to obtain the purified carboxylic acid product. We also attempted to use silicon-based protecting groups, such as trimethylsilyl (TMS) or *tert*butyldimethylsilyl (TBS), to protect the carboxyl group, but the corresponding products were similarly unstable, and it was impossible to obtain purified products.

The shortcomings and unsuccessful attempts in the above synthetic routes have hindered the commercial production of this type of ADC drugs. Therefore, there is a need to explore a more efficient and rational preparation route, along with stable and easily purifiable intermediates, to address this problem.

### SUMMARY

The technical problem to be solved by the present disclosure is to overcome the defects that the intermediates in the existing preparation methods of the linker-drug conjugates represented by formula **LE14** are difficult to purify resulting in the low purity of the prepared **LE14,** and the DXd derivative is expensive, thereby providing a new preparation method for the linker-drug conjugate **LE14** and an intermediate thereof. The preparation method of the present disclosure directly uses exotecan or a mesylate thereof as the payload source, and reacts with the new intermediate **II** of the present disclosure to form a linker-drug conjugate compound **I,** and finally obtains the final product **LE14** through subsequent process reactions. The preparation method of the present disclosure has one or more of the following advantages: The purified carboxylic acid intermediate **II** product can be stably obtained and used as the starting material for process production, which significantly reduces the process steps and also reduces the impurity content of the final product **LE14;** in addition, the more economical exotecan or a mesylate thereof is used as the key raw material in the process, which greatly reduces the production cost.

The structure of the antibody-drug conjugate **LE14** involved in the present disclosure is as follows:

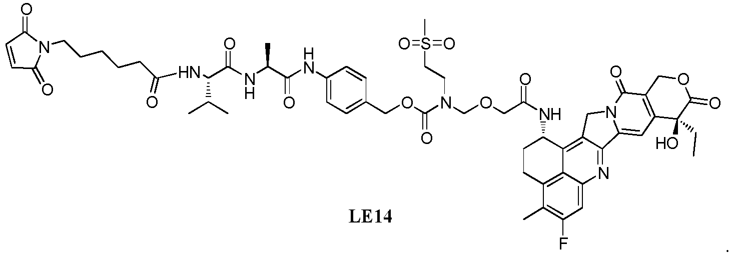

The present disclosure mainly solves the above technical problems through the following technical solutions.

The present disclosure provides a preparation method for a compound of formula **I,** comprising the following step: subjecting an intermediate compound of formula **II** and a compound of formula **III** or a mesylate thereof to an amide condensation reaction in the presence of a condensing agent, a base, and a solvent to obtain the compound of formula **I;**

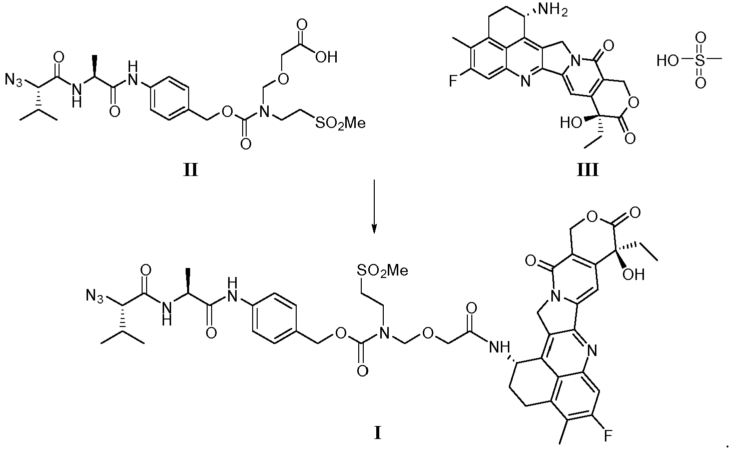

In the preparation method for the compound of formula I, the reaction conditions (such as solvent amount, feeding order, feeding method, and feeding time) may be conventional conditions for such reactions in the art. In the present disclosure, the following schemes are preferred:

In some embodiments, in the preparation method for the compound of formula **I,** materials for the reaction are the compound of formula **II,** the compound of formula **III** or the mesylate thereof, the condensing agent, the base, and the solvent.

In some embodiments, in the preparation method for the compound of formula **I,** the amide condensation reaction further comprises the following specific steps: dissolving the compound of formula **II** in the solvent, adding the condensing agent, and adding the compound of formula **III** or the mesylate thereof and the base immediately or after reacting for a period of time.

In some embodiments, in the preparation method for the compound of formula **I,** the reaction is protected from light throughout the entire process.

In some embodiments, in the preparation method for the compound of formula **I,** the molar ratio of the compound of formula **III** to the compound of formula **II** may be 0.8 to 1.5, preferably 0.9 to 1.2, more preferably 0.9 to 1.0, and most preferably 0.9.

In some embodiments, in the preparation method for the compound of formula **I,** the condensing agent may be one or a mixture of any two or more of 4-(4,6-dimethoxy-1,3,5-triazin-2-yl)-4-methylmorpholinium chloride (DMTMM), diethyl cyanophosphonate (DECP), diphenylphosphoryl azide (DPPA), 1-ethyl-(3-dimethylaminopropyl)carbodiimide hydrochloride (EDCI), 1-hydroxybenzotriazole (HOBt), 2-(7-azabenzotriazol-1-yl)-*N,N,N',N*'-tetramethyluronium hexafluorophosphate (HATU), *O*-(benzotriazol-1-yl)-*N,N,N',N*'-tetramethyluronium hexafluorophosphate (HBTU), *O*-(6-chlorobenzotriazol-1-yl)-*N,N,N',N*'-tetramethyluronium hexafluorophosphate (HCTU), 2-(1*H*-benzotriazol-1-yl)-1,1,3,3-tetramethyluronium tetrafluoroborate (TBTU), 2-succinimido-1,1,3,3-tetramethyluronium tetrafluoroborate (TSTU), and 2-(5-norborene-2,3-dicarboximido)-1,1,3,3-tetramethyluronium tetrafluoroborate (TNTU). Preferably, the condensing agent may be 4-(4,6-dimethoxy-1,3,5-triazin-2-yl)-4-methylmorpholinium chloride.

In some embodiments, in the preparation method for the compound of formula **I,** the molar ratio of the condensing agent to the compound of formula II may be 1 to 3, preferably 1 to 1.5, and more preferably 1.5.

In some embodiments, in the preparation method for the compound of formula I, the base may be a conventional base for such reactions in the art, and may be an organic base, an inorganic base, or a mixture thereof, preferably an organic base; wherein the organic base is preferably one or a mixture of any two or more of *N,N-*diisopropylethylamine, triethylamine, and pyridine, further preferably *N,N-*diisopropylethylamine; the inorganic base is preferably one or a mixture of any two or more of alkali metal hydroxides, alkali metal carbonates, and alkali metal phosphates, further preferably one or a mixture of any two or more of sodium bicarbonate, sodium carbonate, potassium carbonate, cesium carbonate, sodium hydroxide, and potassium hydroxide.

In some embodiments, in the preparation method for the compound of formula **I,** the molar ratio of the base to the compound of formula **II** may be 1 to 10, preferably 1 to 6, more preferably 1 to 3, and most preferably 1.5.

In some embodiments, in the preparation method for the compound of formula **I,** the solvent may be one or a mixture of any two or more of *N*,*N*-dimethylformamide, dimethyl sulfoxide, tetrahydrofuran, and 1,4-dioxane, preferably *N*,*N*-dimethylformamide.

In some embodiments, in the preparation method for the compound of formula **I,** the temperature of the condensation reaction may be 20°C to 50°C, preferably 20°C to 30°C.

In some embodiments, in the preparation method for the compound of formula **I,** the condensation reaction is preferably carried out under the protection of an inert gas, such as in a nitrogen or helium atmosphere.

In some embodiments, in the preparation method for the compound of formula **I,** the reaction progress of the condensation reaction can be monitored by conventional testing methods in the art (such as TLC, GC, HPLC, or NMR), and the reaction endpoint is generally when the compound of formula **III** is no longer detected.

In some embodiments, in the preparation method for the compound of formula **I,** the time of the condensation reaction may be 2 to 8 hours, preferably 3 to 5 hours, and more preferably 2 to 4 hours.

In some embodiments, the preparation method for the compound of formula **I** may further comprise the following post-treatment step after the completion of the condensation reaction: concentrating the reaction mixture to obtain a crude product of the compound of formula **I;** preferably, purifying the crude product of the compound of formula **I** by silica gel column chromatography to obtain a product of the compound of formula **I;** more preferably, the eluent for the silica gel column chromatography may be a mixed solvent of chloroform and methanol, wherein the volume ratio of chloroform to methanol is (100 to 10):1, and further preferably 10:1.

The preparation method for the compound of formula **I** may further comprise a preparation method for the compound of formula **II,** which may comprise the following step: subjecting a compound of formula **IV** to a deprotection reaction in the presence of a deprotecting agent and in a solvent to obtain the compound of formula **II;** 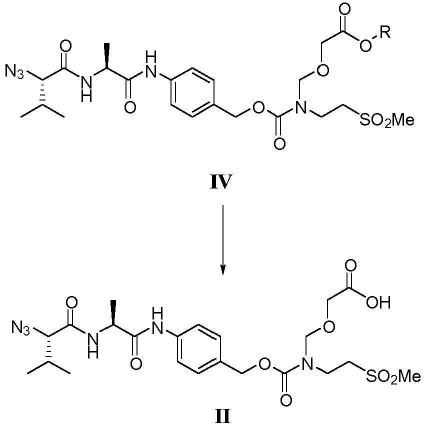 wherein R is C₁-C₆ alkyl substituted by -Si(C₁-C₆)₃.

In some embodiments, in the preparation method for the compound of formula **II,** the C₁-C₆ alkyl substituted by -Si(C₁-C₆)₃ may be trimethylsilylethyl or *tert-*butyldimethylsilylethyl; preferably, the C₁-C₆ alkyl substituted by -Si(C₁-C₆)₃ is trimethylsilylethyl.

In some embodiments, in the preparation method for the compound of formula **II,** materials for the reaction are the compound of formula **IV,** the deprotecting agent, and the solvent.

In some embodiments, in the preparation method for the compound of formula **II,** the deprotecting agent may be a fluorinating reagent. The fluorinating reagent may be tetrabutylammonium fluoride, tetramethylammonium fluoride, tetrabutylammonium fluoride/acetic acid, pyridine hydrofluoride complex, tert-butylammonium fluoride, *tert-*butylammonium fluoride/acetic acid, tetraethylammonium fluoride, or a commercially available tetramethylammonium fluoride/tetrahydrofuran solution, tetraethylammonium fluoride/tetrahydrofuran solution, tetrabutylammonium fluoride/tetrahydrofuran solution, or one or more of silicon tetrafluoride, potassium fluoride, sodium fluoride, lithium fluoride, and cesium fluoride. Preferably, the fluorinating reagent is a 1 M tetrabutylammonium fluoride/tetrahydrofuran solution or potassium fluoride.

In some embodiments, in the preparation method for the compound of formula **II,** the molar ratio of the deprotecting agent to the compound of formula **IV** may be 1 to 5, preferably 1 to 3, more preferably 1.1 to 2.0, and most preferably 1.5.

In some embodiments, in the preparation method for the compound of formula **II,** the solvent may be one or a mixture of any two or more of purified water, *N,N*-dimethylformamide, dimethyl sulfoxide, tetrahydrofuran, and 1,4-dioxane, preferably *N*,*N*-dimethylformamide or tetrahydrofuran, and most preferably *N*,*N*-dimethylformamide.

In certain embodiments, the reaction conditions (*e.g.,* solvent amount, solvent type, feeding order, feeding method, and feeding time) in the preparation method for the compound of formula **II** may be conventional conditions for such reactions in the art, which can be adjusted based on the type of different deprotection groups. For example, the following steps may be comprised: dissolving the compound of formula **IV** in the solvent, adding the deprotecting agent, and initiating the reaction by heating; or dissolving the deprotecting agent in the solvent, adding the compound of formula **IV,** and initiating the reaction by heating.

In some embodiments, in the preparation method for the compound of formula **II,** the temperature of the deprotection reaction is 20°C to 80°C, preferably 30°C to 70°C, more preferably 40°C to 70°C, even more preferably 40°C to 60°C, and most preferably 60°C.

In some embodiments, in the preparation method for the compound of formula **II,** the deprotection reaction is preferably carried out under the protection of an inert gas, such as in a nitrogen or helium atmosphere.

In some embodiments, in the preparation method for the compound of formula **II,** the reaction progress of the deprotection reaction can be monitored by conventional testing methods in the art (such as TLC, GC, HPLC, or NMR), and the reaction endpoint is generally when the compound of formula **IV** is no longer detected.

In some embodiments, in the preparation method for the compound of formula **II,** the time of the deprotection reaction may be 4 to 24 hours, preferably 5 to 16 hours, and more preferably 5 to 8 hours.

In some embodiments, the preparation method for the compound of formula **II** may further comprise a post-treatment step after the completion of the reaction: concentrating the reaction mixture to obtain a crude product of the compound of formula **II**;\ preferably, purifying the crude product of the compound of formula **II** by silica gel column chromatography to obtain a product of the compound of formula **II**; more preferably, the eluent for the silica gel column chromatography is a mixed solvent of dichloromethane and methanol, wherein the volume ratio of dichloromethane to methanol may be (100 to 1):1, preferably (10 to 1):1.

The preparation method for the compound of formula **I** may further comprise a preparation method for the compound of formula **IV,** which may comprise the following step: subjecting a compound of formula **VI** to an etherification reaction with reagent **V** in a solvent and in the presence of a base to obtain the compound of formula **IV**; 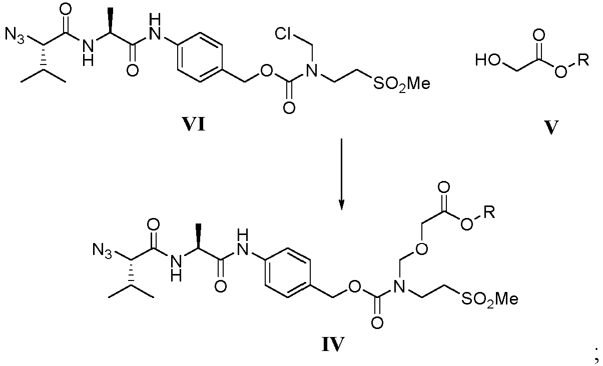 wherein R is as described above.

In some embodiments, in the preparation method for the compound of formula **IV,** materials for the reaction are the compound of formula **VI,** the reagent **V,** the base, and the solvent.

In some embodiments, in the preparation method for the compound of formula **IV,** the molar ratio of the reagent **V** to the compound of formula **VI** may be 1 to 5, preferably 1 to 3, more preferably 1.1 to 1.6, and most preferably 1.5.

In some embodiments, in the preparation method for the compound of formula **IV,** the base may be an organic base, an inorganic base, or a mixture thereof, preferably an organic base; wherein the organic base is preferably one or a mixture of any two or more of potassium tert-butoxide, triethylamine, 4-dimethylaminopyridine, pyridine, and pempidine, more preferably pempidine; the inorganic base is preferably one or a mixture of any two or more of alkali metal hydroxides, alkali metal carbonates, and alkali metal phosphates.

In some embodiments, in the preparation method for the compound of formula **IV,** the molar ratio of the base to the compound of formula **VI** may be 1 to 5, preferably 1.2 to 4, more preferably 1.5 to 3, and most preferably 1.5.

In some embodiments, in the preparation method for the compound of formula **IV,** the solvent is one or a mixture of any two or more of *N,N*-dimethylformamide, dimethyl sulfoxide, tetrahydrofuran, and 1,4-dioxane, preferably tetrahydrofuran or 1,4-dioxane, and more preferably tetrahydrofuran.

In some embodiments, in the preparation method for the compound of formula **IV,** the reaction conditions (such as solvent amount, feeding order, feeding method, and feeding time) may be conventional conditions for such reactions in the art, which can be adjusted according to the types of different alkaline reagents. For example, the following steps may be comprised: dissolving the compound of formula **VI** in the solvent, adding the base, and adding reagent **V** immediately or after reacting for a period of time.

In some embodiments, in the preparation method for the compound of formula **IV,** the temperature of the etherification reaction may be 0°C to 80°C, preferably 40°C to 70°C, and most preferably 60°C.

In some embodiments, in the preparation method for the compound of formula **IV,** the etherification reaction is preferably carried out under the protection of an inert gas, such as in a nitrogen or helium atmosphere.

In some embodiments, in the preparation method for the compound of formula **IV,** the reaction progress of the etherification reaction can be monitored by conventional testing methods in the art (such as TLC, GC, HPLC, or NMR), and the reaction endpoint is generally when the compound of formula **VI** is no longer detected.

In some embodiments, in the preparation method for the compound of formula **IV,** the time of the etherification reaction may be 3 to 24 hours, preferably 3 to 12 hours, and more preferably 4 to 8 hours.

In some embodiments, the preparation method for the compound of formula **IV** may further comprise post-treatment steps after the completion of the reaction: extracting the reaction mixture and concentrating the organic phase to obtain a crude product of the compound of formula **IV,** or directly concentrating the reaction mixture to obtain a crude product of the compound of formula **IV;** preferably, purifying the crude product of the compound of formula **IV** by silica gel column chromatography to obtain a product of the compound of formula **IV;** more preferably, the eluent for the silica gel column chromatography is a mixed solution of *n-*heptane and ethyl acetate, wherein the volume ratio of *n*-heptane to ethyl acetate may be (20 to 1):1, preferably (10 to 1):1.

The preparation method for the compound of formula **I** may further comprise a preparation method for the compound of formula **VI,** which may comprise the following step: subjecting a compound of formula **VII** to a substitution reaction with paraformaldehyde and trimethylsilyl chloride in a solvent to obtain the compound of formula **VI;** 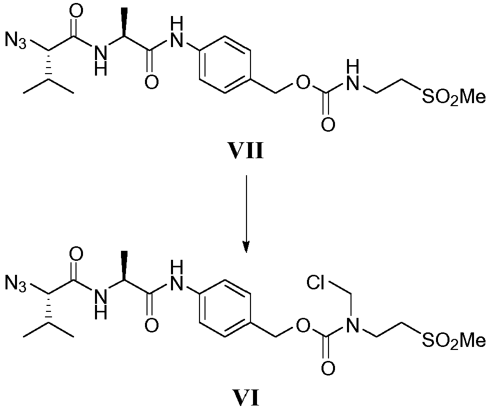 wherein the compound of formula **VII** is an azido-containing polypeptide compound, which is commercially available or synthesized in-house.

In some embodiments, in the preparation method for the compound of formula **VI,** materials for the reaction are the compound of formula **VII,** paraformaldehyde, trimethylsilyl chloride, and the solvent.

In some embodiments, in the preparation method for the compound of formula **VI,** the molar ratio of paraformaldehyde to the compound of formula **VII** may be 1 to 10, preferably 1 to 5, more preferably 1.3 to 3.0, and most preferably 1.3.

In some embodiments, in the preparation method for the compound of formula **VI,** the molar ratio of trimethylsilyl chloride to the compound of formula **VII** may be 1 to 5, preferably 2 to 4, more preferably 2 to 3, and most preferably 2.5.

In some embodiments, in the preparation method for the compound of formula **VI,** the solvent may be one or a mixture of any two or more of *N*,*N*-dimethylformamide, dimethyl sulfoxide, tetrahydrofuran, and 1,4-dioxane, preferably tetrahydrofuran or 1,4-dioxane, and more preferably 1,4-dioxane.

In some embodiments, in the preparation method for the compound of formula **VI,** the temperature of the substitution reaction is -10°C to 50°C, preferably 15°C to 35°C, and more preferably 18°C to 25°C.

In some embodiments, in the preparation method for the compound of formula **VI,** the substitution reaction is preferably carried out under the protection of an inert gas, such as in a nitrogen or helium atmosphere.

In some embodiments, in the preparation method for the compound of formula **VI,** the reaction progress of the substitution reaction can be monitored by conventional testing methods in the art (such as TLC, GC, HPLC, or NMR), and the reaction endpoint is generally when the compound of formula **VII** (using methanol derivatization) is no longer detected.

In some embodiments, in the preparation method for the compound of formula **VI,** the time of the substitution reaction may be 3 to 24 hours, preferably 6 to 18 hours, and more preferably 8 to 16 hours.

In some embodiments, the preparation method for the compound of formula **VI** may further comprise post-treatment steps after the completion of the reaction: subjecting the reaction mixture to solid-liquid separation (if solids are present) or omitting solid-liquid separation, followed by concentrating the organic phase to obtain the compound of formula **VI;** preferably, directly reacting the crude product of the compound of formula **VI** obtained after concentration with reagent **V.**

The present disclosure also provides a preparation method for a compound of formula **VIII,** comprising the following step: subjecting a compound of formula I to a reduction reaction with a reducing agent in the presence of a solvent and an acid buffer to obtain the compound of formula **VIII**;

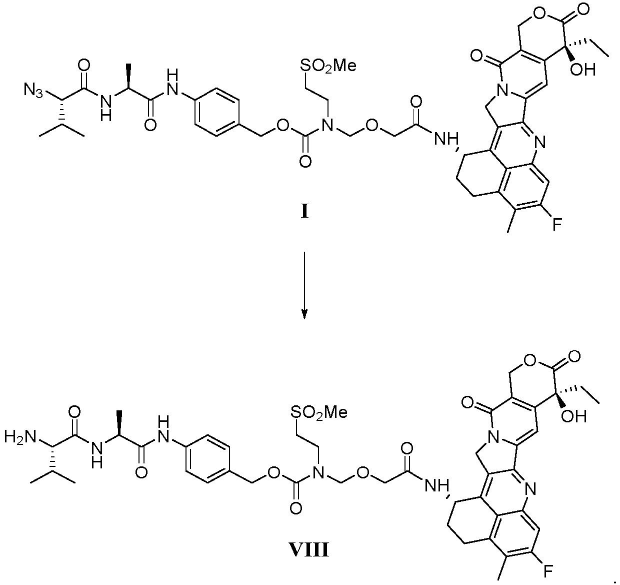

In some embodiments, in the preparation method for the compound of formula **VIII**, materials for the reaction are the compound of formula I, the reducing agent, the acid buffer, and the solvent.

In some embodiments, in the preparation method for the compound of formula **VIII**, the reducing agent may be triphenylphosphine, tri-tert-butylphosphine, or trimethylphosphine, or a commercially available triphenylphosphine/tetrahydrofuran solution, tri-*tert-*butylphosphine/tetrahydrofuran solution, or trimethylphosphine/tetrahydrofuran solution, preferably a trimethylphosphine/tetrahydrofuran solution, and more preferably a 1 M trimethylphosphine/tetrahydrofuran solution.

In some embodiments, in the preparation method for the compound of formula **VIII,** the acid buffer may be a sodium acetate buffer or a sodium formate buffer, preferably a sodium acetate buffer; the pH of the acid buffer is preferably 4.0 to 6.0, more preferably 4.5 to 5.5, and even more preferably 5.0.

In some embodiments, in the preparation method for the compound of formula **VIII,** the molar ratio of the reducing agent to the compound of formula I may be 1.0 to 3.0, preferably 1.2 to 1.8, and more preferably 1.5.

In some embodiments, in the preparation method for the compound of formula **VIII,** the volume-to-mass ratio of the acid buffer to the compound of formula I may be 2 to 10 mL/g, preferably 5 to 10 mL/g, more preferably 7 to 8 mL/g, and most preferably 7.7 mL/g.

In some embodiments, in the preparation method for the compound of formula **VIII,** the solvent is an ether solvent, such as one or a mixture of any two or more of tetrahydrofuran, diethyl ether, 1,4-dioxane, anisole, and methyl tert-butyl ether, and is further preferably tetrahydrofuran.

In some embodiments, in the preparation method for the compound of formula **VIII,** the temperature of the reduction reaction may be 0°C to 20°C, preferably 0°C to 10°C.

In some embodiments, in the preparation method for the compound of formula **VIII,** the reaction progress can be monitored by conventional testing methods in the art (such as TLC, GC, HPLC, or NMR), and the reaction endpoint is generally when the compound of formula I is no longer detected.

In some embodiments, in the preparation method for the compound of formula **VIII,** the time of the reaction may be 0.5 to 5 hours, preferably 0.5 to 2.5 hours, and more preferably 0.5 to 1.0 hours.

In some embodiments, the preparation method for the compound of formula **VIII** may further comprise post-treatment steps after the completion of the reaction: extracting the reaction mixture and concentrating the organic phase to obtain a crude product of the compound of formula **VIII,** or directly concentrating the reaction mixture to obtain a crude product of the compound of formula **VIII**; preferably, purifying the crude product of the compound of formula **VIII** by silica gel column chromatography to obtain a product of the compound of formula **VIII**; more preferably, the eluent for the silica gel column chromatography is a mixed solvent of dichloromethane and methanol, wherein the volume ratio of dichloromethane to methanol may be (100 to 1):1, preferably (10 to 1):1.

The present disclosure also provides a preparation method for compound **LE14,** comprising the following step: subjecting a compound of formula **VIII** and *N*-succinimidyl 6-maleimidohexanoate to a coupling reaction in a solvent to obtain the compound **LE14,** *i.e.,* linker-drug conjugate **LE14.**

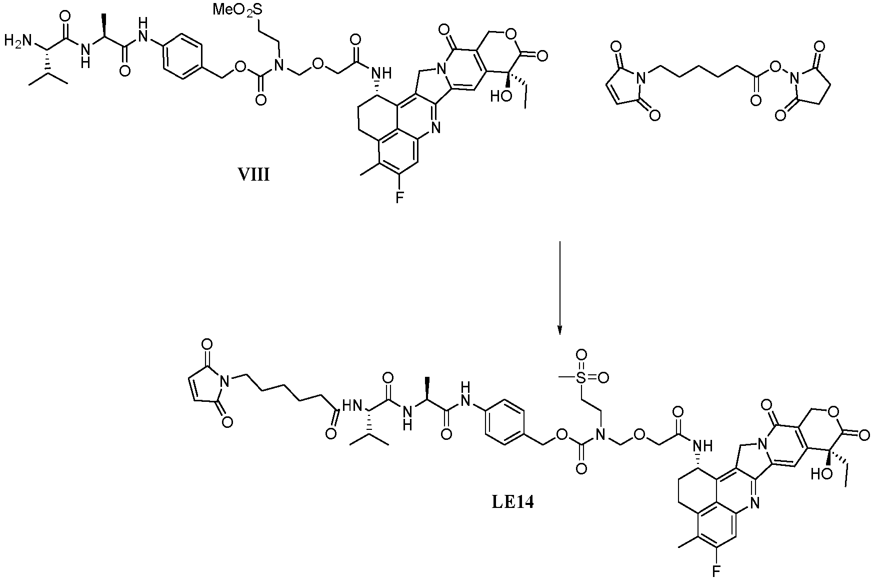

In some embodiments, in the preparation method for the compound **LE14,** materials for the reaction are the compound of formula **VIII,** *N*-succinimidyl 6-maleimidohexanoate, and the solvent.

In some embodiments, in the preparation method for the compound **LE14,** the molar ratio of *N*-succinimidyl 6-maleimidohexanoate to the compound of formula **VIII** may be 1 to 5, preferably 1 to 3, more preferably 1.2 to 2.0, and most preferably 2.0.

In some embodiments, in the preparation method for the compound **LE14,** the solvent may be one or a mixture of any two or more of amide solvents, chlorinated alkane solvents, ether solvents, and nitrile solvents, preferably, a chlorinated alkane solvent; wherein the chlorinated alkane solvent is preferably one or a mixture of any two or more of dichloromethane, 1,2-dichloroethane, and chloroform, further preferably dichloromethane.

In some embodiments, in the preparation method for the compound **LE14,** the temperature of the reaction is 0°C to 45°C, preferably 25°C to 40°C, more preferably 30°C to 35°C, and most preferably 35°C.

In some embodiments, in the preparation method for the compound **LE14,** the reaction progress can be monitored by conventional testing methods in the art (such as TLC, GC, HPLC, or NMR), and the reaction endpoint is generally when the compound of formula **VIII** is no longer detected.

In some embodiments, in the preparation method for the compound **LE14,** the time of the reaction may be 3 to 24 hours, preferably 3 to 10 hours, and more preferably 3 to 6 hours.

In some embodiments, the preparation method for the compound **LE14** may further comprise a post-treatment step after the completion of the reaction: concentrating the reaction mixture to obtain a crude product of the compound **LE14;** preferably, purifying the crude product of the compound **LE14** by silica gel column chromatography to obtain a product of the compound **LE14;** more preferably, the eluent for the silica gel column chromatography is a mixed solvent of dichloromethane and methanol, wherein the volume ratio of dichloromethane to methanol may be (100 to 1):1, preferably (10 to 1):1.

In some embodiments, in the preparation method for the compound **LE14,** the compound of formula **VIII** may be prepared by any of the schemes described in the above preparation method for the compound of formula **VIII.**

The present disclosure also provides a compound of formula **II**:

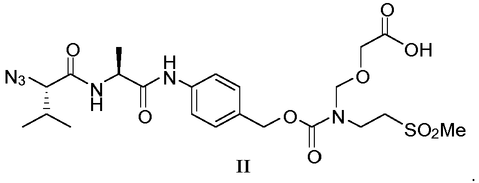

The present disclosure also provides a preparation method for a compound of formula II, comprising the following step: subjecting a compound of formula **IV** to a deprotection reaction in the presence of a deprotecting agent and in a solvent to obtain the compound of formula **II;** 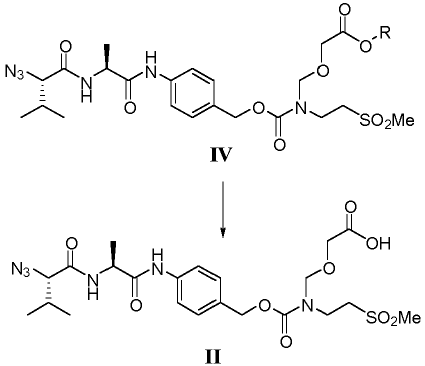 wherein R is C₁-C₆ alkyl substituted by -Si(C₁-C₆)₃.

In the preparation method for the compound of formula **II,** the reaction conditions may be as described above.

The preparation method for the compound of formula **II** may further comprise the preparation method for the compound of formula **IV** as described herein.

### Definition

In the present disclosure, the term "C₁-C₆ alkyl" refers to a saturated linear or branched alkyl group comprising 1 to 6, particularly 1 to 4 carbon atoms, such as methyl, ethyl, *n*-propyl, isopropyl, *n*-butyl, isobutyl, or *tert*-butyl, particularly methyl or ethyl.

In the present disclosure, room temperature refers to 20°C to 30°C.

The English abbreviations and corresponding full names of the compounds involved in the present disclosure are listed in Table 1.

**Table 1**

| | |
|---|---|
| EMCS | *N*-Succinimidyl 6-maleimidohexanoate |
| PMP | Pempidine/1,2,2,6,6-Pentamethylpiperidine |
| DMF | *N*,*N*-Dimethylformamide |
| DMSO | Dimethyl sulfoxide |
| DMTMM | 4-(4,6-Dimethoxy-1,3,5-triazin-2-yl)-4-methylmorpholinium chloride |
| TLC | Thin-layer chromatography |
| GC | Gas chromatography |
| HPLC | High-performance liquid chromatography |
| NMR | Nuclear magnetic resonance |
| DMAP | 4-Dimethylaminopyridine |
| TBAF | Tetrabutylammonium fluoride |
| TMAF | Tetramethylammonium fluoride |
| DIEA | *N,N-Diisopropylethylamine* |
| TEAF | Tetraethylammonium fluoride |
| HCHO | Paraformaldehyde |
| PMe3 | Trimethylphosphine |
| TMSCl | Trimethylsilyl chloride |
| Exatecan | Exatecan |

The above preferred conditions can be combined arbitrarily to obtain preferred embodiments of the present disclosure without violating common knowledge in the art.

The reagents and starting materials used in the present disclosure are commercially available or synthesized in-house.

The positive and progressive effects of the present disclosure are as follows: The present disclosure provides a novel preparation method for a linker-drug conjugate intermediate represented by formula **I.** Firstly, this preparation method can stably provide a pure intermediate **II,** with a yield of up to 89.1% and a purity of up to 90.16%. Compared to the methods for preparing the carboxylic acid intermediate by removing the *tert-butyl* group in Route 1 and Route 2, both the yield and purity are significantly improved.

Secondly, using the purified intermediate **II** for the condensation reaction can significantly enhance the purity of intermediate **I** and reduce impurity generation. Continuing the subsequent synthesis steps can also significantly improve the purity of the final product **LE14,** reducing the maximum single impurity content in the final product **LE14** to 0.1% or less.

Finally, using intermediate **II** as the starting material for the commercial production process can simplify the production steps. At the same time, the condensation of intermediate **II** with commercially available exatecan mesylate avoids the use of costly DXd materials, significantly reducing production costs.

### DETAILED DESCRIPTION OF THE PREFERRED EMBODIMENT

The present disclosure will be further described below with reference to examples, but the present disclosure is not therefore limited to the scope of the examples. Experimental methods without specific conditions in the following examples are selected according to conventional methods and conditions, or according to commercial instructions.

In the following examples,
the mass spectrometry was performed using a Waters Acquity Xevo G2-XS QTof UPLC/MS ultra-high-performance liquid chromatography high-resolution mass spectrometry system;
¹H-NMR was performed using a Bruker AVANCE III 400 MHz NMR spectrometer or a Bruker AVANCE III HD 300 MHz NMR spectrometer;
HPLC detection conditions: The instrument was Agilent 187260, the chromatographic column was Agilent AdvanceBio Peptide Map, 3.5 µm, 2.1 × 250 mm, the detection wavelength was 254 nm, and the mobile phase gradient setting shown in Table 2 was adopted, wherein phase A was 0.01 mol/L potassium dihydrogen phosphate aqueous solution (pH = 5.0), phase B was 10% methanol-acetonitrile solution, and the flow rate was 1.0 mL/min.

**Table 2. HPLC Detection Mobile Phase Gradient Settings**

| Time (min) | Mobile Phase A% | Mobile Phase B% |
|---|---|---|
| 0.00 | 80.0 | 20.0 |
| 5.00 | 60.0 | 40.0 |
| 35.00 | 60.0 | 40.0 |
| 40.00 | 30.0 | 70.0 |
| 46.00 | 30.0 | 70.0 |
| 46.10 | 80.0 | 20.0 |
| 60.00 | 80.0 | 20.0 |

Conventional column chromatography conditions were: The filler was 200 to 400 mesh amorphous silica gel, and the column chromatography was performed at room temperature.

In the following examples, room temperature refers to 20 to 30°C.

### Example 1 Synthesis of Compound LE14

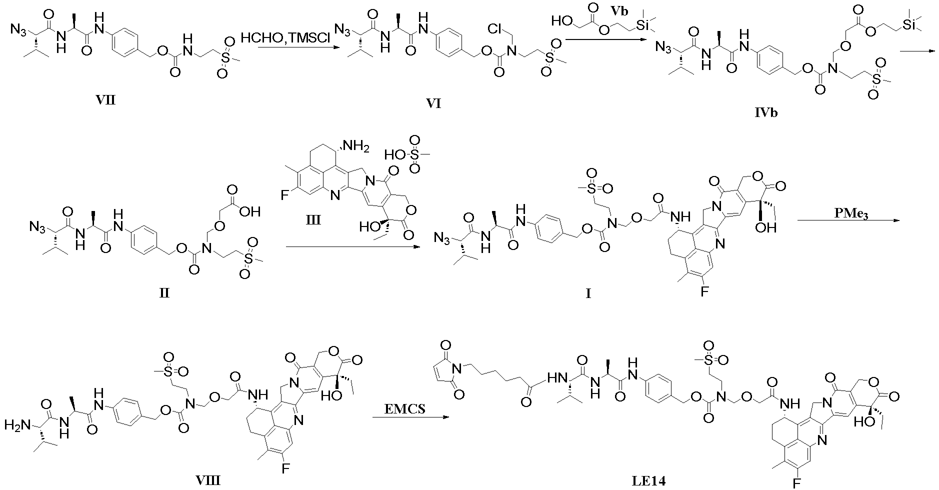

### Step 1: Synthesis of Compound of Formula VI

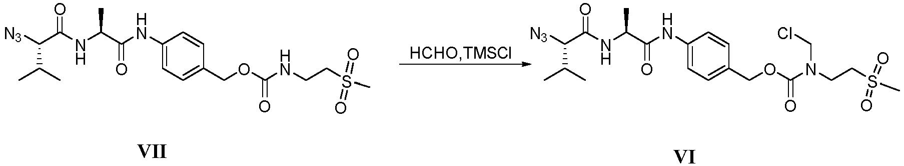

A compound of formula **VII** (25.0 g, 53.36 mmol) and paraformaldehyde (2.09 g, 69.37 mmol) were mixed and dissolved in 250.0 mL of anhydrous 1,4-dioxane. Trimethylsilyl chloride (14.43 g, 133.40 mmol) was slowly added, and the reaction was carried out at 18 to 25°C for 15.0 hours to obtain a crude solution of the compound of formula **VI.** The reaction mixture was sampled for in-process control. After the reaction was completed, the reaction mixture was filtered, and the filtrate was concentrated to obtain a crude product of the compound of formula **VI** (28.40 g). (Used directly for the next reaction)

### Step 2: Synthesis of Compound of Formula IVb

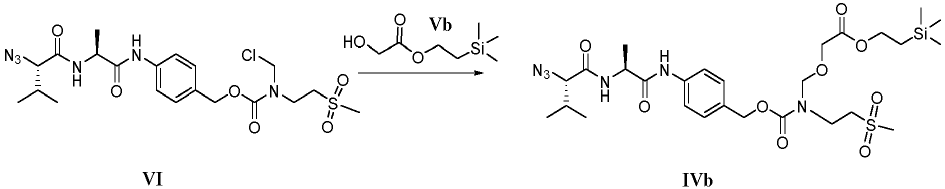

The resulting crude product of the compound of formula **VI** (28.40 g, 53.36 mmol calculated based on the theoretical yield) was dissolved in 250.0 mL of anhydrous tetrahydrofuran, followed by the addition of pempidine (12.43 g, 80.05 mmol) and a compound of formula **Vb** (14.12 g, 80.05 mmol). The reaction was heated to 60°C under a nitrogen atmosphere and carried out for 4.0 to 8.0 hours under this condition. After the reaction of the starting materials was completed, the tetrahydrofuran was removed by concentration under reduced pressure. Then, ethyl acetate and saturated brine were added for extraction. The resulting organic phase was dried and concentrated under reduced pressure. The resulting crude product was purified by silica gel column chromatography (*n*-heptane: ethyl acetate = 10:1 to 1:1 (v/v)) to obtain the compound of formula **IVb** (17.88 g, purity: 92.81%, combined yield of step 1 and step 2: 51.0%).
ESI-MS m/z: 657.4 (M+H);
¹H NMR (400 MHz, DMSO-*d*₆) δ 10.12 (s, 1H), 8.49 (d, *J =* 7.0 Hz, 1H), 7.59 (d, *J =* 8.6 Hz, 2H), 7.42-7.21 (m, 2H), 5.15-4.97 (m, 2H), 4.85 (s, 2H), 4.46 (p, *J =* 7.0 Hz, 1H), 4.12 (dd, *J =* 29.5, 10.7 Hz, 4H), 3.68 (dd, *J=* 8.7, 6.0 Hz, 2H), 3.49 (d, *J =* 8.2 Hz, 1H), 3.40 (t, *J* = 7.3 Hz, 2H), 2.97 (d, *J* = 26.2 Hz, 3H), 1.33 (d, *J=* 7.1 Hz, 3H), 1.29-1.18 (m, 2H), 0.93 (dd, *J=* 9.6, 6.6 Hz, 6H), 0.87-0.82 (m, 1H), 0.15 (s, 9H).

### Step 3: Synthesis of Compound of Formula II

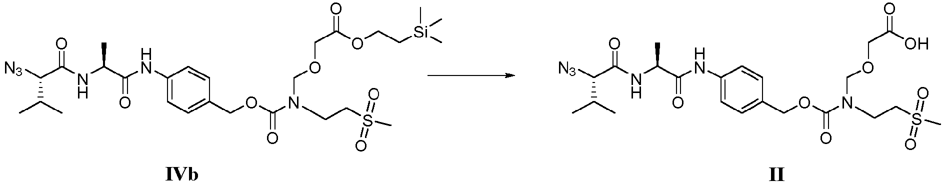

### Method 1: Deprotection Using Potassium Fluoride Reagent

The compound of formula **IVb** (5.0 g, 7.62 mmol) was dissolved in 50.0 mL of DMF, and potassium fluoride (0.67 g, 11.42 mmol) was added at room temperature to form a mixed solution. The reaction was heated to 60°C under a nitrogen atmosphere and carried out for 5.0 to 8.0 hours under this condition. After the reaction of the starting materials was completed, the solvent was removed by concentration under reduced pressure. The resulting crude product was purified by silica gel column chromatography (dichloromethane: methanol = 10:1 to 1:1 (v/v)) to obtain the compound of formula **II** (3.78 g, yield: 89.1%).
ESI-MS m/z: 556.0 (M-H);
¹H NMR (400 MHz, DMSO-*d*₆) δ ppm 10.57 (d, *J =* 38.3 Hz, 1H), 9.36 (d, *J =* 94.5 Hz, 1H), 7.61 (d, *J =* 8.5 Hz, 2H), 7.28 (d, *J =* 7.2 Hz, 2H), 5.01 (d, *J =* 10.8 Hz, 2H), 4.83 (s, 2H), 4.38 (s, 1H), 4.17 (s, 1H), 3.71 (s, 2H), 3.54 (s, 2H), 3.48 (d, *J =* 8.3 Hz, 1H), 3.44 (s, 1H), 3.14 (s, 2H), 2.95 (d, *J =* 22.9 Hz, 3H), 1.32 (d, *J =* 7.2 Hz, 3H), 0.89 (dd, *J =* 14.7, 6.7 Hz, 6H).

### Method 2: Deprotection Using Sodium Fluoride Reagent

The operation steps and reaction conditions were the same as those of method 1, except that potassium fluoride was replaced by an equal molar amount of sodium fluoride, and the amounts of raw materials and solvents used were the same as those of method 1. The compound of formula **II** (2.20 g, yield: 52.0%) was obtained after purification.

### Method 3: Deprotection Using Cesium Fluoride Reagent

The operation steps and reaction conditions were the same as those of method 1, except that potassium fluoride was replaced by an equal molar amount of cesium fluoride, and the amounts of raw materials and solvents used were the same as those of method 1. The compound of formula **II** (1.84 g, yield: 43.5%) was obtained after purification.

### Method 4: Deprotection Using Tetrabutylammonium Fluoride Reagent

The compound of formula **IVb** (5.0 g, 7.62 mmol) was dissolved in tetrahydrofuran (50.0 mL), and a 1.0 M tetrabutylammonium fluoride solution in tetrahydrofuran (11.5 mL, 11.43 mmol) was added at room temperature. The reaction was slowly heated to 40°C under a nitrogen atmosphere and carried out for 8.0 hours under this condition. The solvent was removed by concentration under reduced pressure. The resulting crude product was purified by silica gel column chromatography (dichloromethane: methanol = 10:1 to 1:1 (v/v)) to obtain the compound of formula **II** (2.99 g, yield: 70.3%).

ESI-MS m/z: 557.5 (M+H).

### Step 4: Synthesis of Compound of Formula I

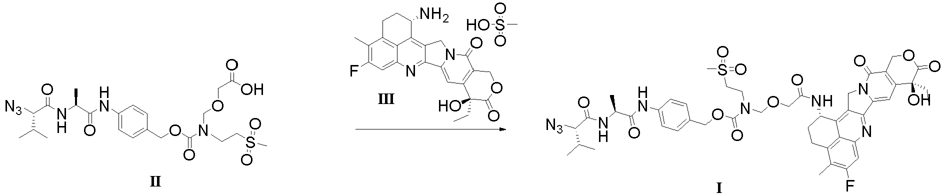

The compound of formula **II** (2.79 g, 5.00 mmol) was dissolved in 20.0 mL of anhydrous DMF, and 4-(4,6-dimethoxy-1,3,5-triazin-2-yl)-4-methylmorpholinium chloride (DMTMM) (2.08 g, 7.50 mmol) was added, and then the reaction was carried out at room temperature for 1.0 hour. Then, *N*,*N*-diisopropylethylamine (1.30 mL, 7.50 mmol) and a compound of formula **III** (exatecan mesylate) (2.40 g, 4.50 mmol) were added thereto, and the reaction was carried out for another 1.5 to 2.0 hours. After the reaction of the starting materials was completed, the solvent was removed by concentration under reduced pressure. The resulting crude product was purified by silica gel column chromatography (chloroform: methanol = 10:1 (v/v)) to obtain the compound of formula **I** (3.44 g, purity: 91.30%, yield: 70.5%).

ESI-MS m/z: 974.5 (M+H).

### Step 5: Synthesis of Compound of Formula VIII

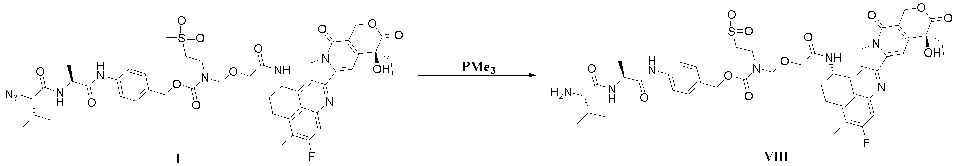

A mixture of 2.1 mL of a 1.0 mol/L trimethylphosphine solution in tetrahydrofuran (2.1 mL, 2.01 mmol) and 5.0 mL of THF was added dropwise to 10.0 mL of sodium acetate buffer (pH = 5.0), and the reaction was cooled to 0 to 5°C under a nitrogen atmosphere. Subsequently, a solution of the compound of formula **I** (1.30 g, 1.34 mmol) dissolved in 5.0 mL of tetrahydrofuran was slowly added dropwise to the reaction system, and the reaction was carried out for another 0.5 to 1.0 hour at 0 to 10°C. After the reaction of the starting materials was completed, the tetrahydrofuran solvent was removed by concentration under reduced pressure. The residue was extracted with dichloromethane, and the resulting organic phase was dried and then evaporated under reduced pressure to remove the solvent. The resulting crude product was purified by silica gel column chromatography (dichloromethane: methanol = 10:1 to 1:1 (v/v)) to obtain the compound of formula **VIII** (980 mg, purity: 89.75%, yield: 77.5%).

ESI-MS m/z: 948.7 (M+H);

### Step 6: Synthesis of Compound LE14

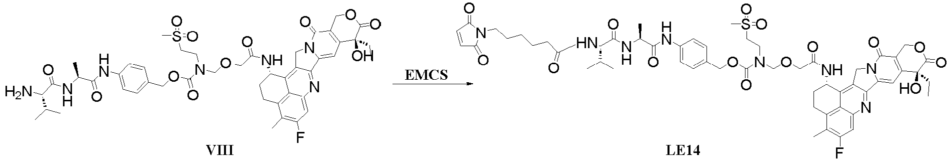

The compound of formula **VIII** (0.98 g, 1.04 mmol) was dissolved in 30.0 mL of anhydrous dichloromethane, and N-succinimidyl 6-maleimidohexanoate (0.64 g, 2.08 mmol) was added. The reaction was heated to 35°C under a nitrogen atmosphere and carried out for 3 to 6 hours under this condition. After the reaction of the starting materials was completed, the solvent was removed by concentration under reduced pressure. The resulting crude product was purified by silica gel column chromatography (dichloromethane: methanol = 10:1 to 1:1 (v/v)) to obtain compound **LE14** (861 mg, purity: 99.66%, yield: 72.5%).
ESI-MS m/z: 1141.6 (M+H);
¹H NMR (500 MHz, DMSO-*d*₆) δ ppm 9.93 (s, 1H), 8.49 (s, 1H), 8.13 (d, *J* = 6.9 Hz, 1H), 7.76 (d, *J =* 28.7 Hz, 2H), 7.57 (s, 2H), 7.28 (s, 3H), 6.99 (s, 2H), 6.48 (s, 1H), 5.59 (s, 1H), 5.47-5.32 (m, 2H), 5.17 (s, 2H), 5.04-4.79 (m, 4H), 4.38 (d, *J=* 7.1 Hz, 1H), 4.21-4.14 (m, 1H), 4.03 (s, 2H), 3.69 (d, *J =* 2.8 Hz, 2H), 3.37 (dt, *J =* 14.2, 7.7 Hz, 4H), 3.24-3.09 (m, 2H), 2.93 (d, *J* = 37.1 Hz, 3H), 2.36 (t, *J* = 1.8 Hz, 3H), 2.22-2.07 (m, 4H), 2.01-1.77 (m, 3H), 1.52-1.41 (m, 4H), 1.30 (d, *J* = 7.1 Hz, 3H), 1.19 (d, *J* = 7.7 Hz, 2H), 0.84 (d, *J =* 12.0 Hz, 9H).

### Example 2

The other feed materials and reaction conditions remained unchanged, and the crude product of the compound of formula **II** prepared according to the methods of step 1 to step 3 of Example 1 (the reaction mixture of step 3 was directly concentrated without purification) was directly subjected to subsequent reactions, and the operations of step 4 to step 6 of Example 1 were followed to finally obtain the purified compound **LE14** (894 mg, purity: 95.46%, yield: 75.3%).

### Example 3

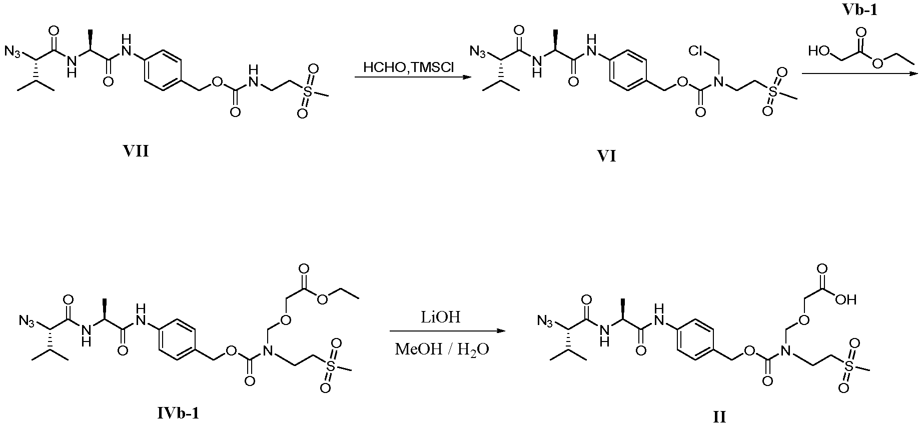

The synthesis steps of **IVb-1** referred to steps 1 and 2 in Example 1, and **Vb** was replaced by **Vb-1** (ethyl glycolate) to obtain a compound of formula **IVb-1** (18.28 g, purity: 94.10%, combined yield of steps 1 and 2: 58.6%).

### Synthesis Steps of Compound of Formula II:

The compound of formula **IVb-1** (5.9 g, 10.00 mmol) was dissolved in 50.0 mL of methanol, a lithium hydroxide aqueous solution (360 mg of lithium hydroxide was dissolved in 10 mL of water) was added at 0 to 10°C. After the addition was completed, the reaction was carried out at 20 to 30°C for 3 to 4 hours. The raw materials were monitored until the reaction was completed, and methanol was removed by concentration under reduced pressure. A 1 mol/L ammonium chloride aqueous solution (15 mL) was added dropwise to the concentrate for neutralization. The resulting mixture was extracted with a dichloromethane/water system, and the organic phase was concentrated to obtain a crude product. The resulting crude product was purified by silica gel column chromatography and the compound of formula **II** was not obtained.

### Example 4 Comprehensive Comparison of Process Routes

### 1. Comparison of Carboxylic Acid Intermediates Prepared via Different Routes

The crude product of carboxylic acid intermediate 1-4 obtained via Route 1 was purified by silica gel column chromatography (chloroform: methanol = 10:1 (v/v)), but the purified intermediate 1-4 was not obtained. The crude product of carboxylic acid intermediate 2-2a obtained via Route 2 was purified by silica gel column chromatography (chloroform: methanol = 10:1 (v/v)), but the purified intermediate 2-2a was not obtained. The carboxylic acid intermediate **II** was successfully obtained in good yield following the method described in Example 1. A summary comparison of the purification results for carboxylic acid intermediates prepared via the three synthetic routes is shown in Table 3 below.

### 2. Comparison of Compound LE14 Prepared via Different Routes

The compound **LE14** synthesized according to Example 1 and Example 2 was compared in purity via high-performance liquid chromatography with the compound **LE14** obtained from Route 1, Route 2, Route 3, Route 4, and Route 5. The results are shown in Table 4 below.

From the data in Table 4, it can be seen that the preparation of compound **LE14** by the method of Example 1 of the present disclosure, due to the change of the protecting group, results in milder deprotection conditions. The resulting carboxylic acid intermediate can stably exist, which on the one hand reduces the content of the maximum single impurity, and on the other hand increases the purity of the compound **LE14** product to 99.5% or more, which is better than the methods of Route 1 to Route 5 (the maximum single impurity content is higher than 0.3%, and the product purity of compound **LE14** is below 98.7%). In Example 2, the compound of formula **II** in step 3 is used directly in subsequent reactions without purification, resulting in a maximum single impurity content higher than 1% and a product purity of only 95.46% for the obtained compound **LE14.** This indicates that the purification of the crude product of the compound of formula **II** in step 3 has a significant impact on the purity and maximum single impurity content of the **LE14** product.

In the present disclosure, the compound of formula **IVb** can stably exist in the crude product of the compound of formula **IVb** obtained by protecting the carboxylic acid with trimethylsilylethyl in step 2 of Example 1. The crude product of the compound of formula **IVb** can be purified by conventional column chromatography. During the purification process, the compound of formula **IVb** remains stable with minimal loss, resulting in the purified compound of formula **IVb.** This effectively prevents the introduction of impurities into subsequent reaction steps.

In the present disclosure, the carboxylic acid intermediate compound of formula **II** can withstand the deprotection conditions of method 1 in step 3, and can stably exist under both the crude product of the compound of formula **II** obtained after deprotection via method 1 in step 3 and conventional column chromatography conditions. The crude product of the compound of formula **II** is purified through conventional column chromatography to obtain a high-purity carboxylic acid intermediate **II** with low impurity content, thereby preventing impurities from being carried over into subsequent reaction steps. As a result, the purity of the final product of compound **LE14** is improved while the maximum single impurity content is reduced, achieving a final product of compound **LE14** with a purity higher than 99.5% and a maximum single impurity content lower than 0.1%.

The liquid phase conditions: phase A: 0.01 mol/L potassium dihydrogen phosphate aqueous solution (pH = 5.0), phase B: 10% methanol-acetonitrile solution; detection wavelength: 254 nm; instrument: Agilent 187260; chromatographic column: Agilent AdvanceBio Peptide Map, 3.5 µm, 2.1 × 250 mm; flow rate: 1.0 mL/min. The gradient settings are shown in Table 2 below.

**Table 2. Mobile Phase Gradient Settings**

| Time (min) | Mobile Phase A% | Mobile Phase B% |
|---|---|---|
| 0.00 | 80.0 | 20.0 |
| 5.00 | 60.0 | 40.0 |
| 35.00 | 60.0 | 40.0 |
| 40.00 | 30.0 | 70.0 |
| 46.00 | 30.0 | 70.0 |
| 46.10 | 80.0 | 20.0 |
| 60.00 | 80.0 | 20.0 |

**Table 3. Comparison of Carboxylic Acid Intermediates (1-4, 2-2a, II) Prepared by Four Different Process Routes**

| Process Route | Purification Situation | |
|---|---|---|
| Route 1 (1-4) | Decomposition occurs during the purification process, and no purified product is obtained. | |
| Route 2 (2-2a) | Decomposition occurs during the purification process, and no purified product is obtained. | |
| Example 3 (**II**) | Decomposition occurs during the purification process, and no purified product is obtained. | |
| Route of the present disclosure Example 1 (**II**) | HPLC purity before purification: 81.14% | Single-step yield after purification: 89.1% |
| | HPLC purity after purification: 90.16% | |

**Table 4. Purity Data Comparison of Final Products of LE14 Using Different Process Routes**

| Route | Payload Source | Product Purity | Maximum Single Impurity Content |
|---|---|---|---|
| Route 1 | Exatecan mesylate | 95.22% | 1.43% |
| Route 2 | Exatecan mesylate | 96.52% | 0.97% |
| Route 3 | DXd | 97.71% | 0.31% |
| Route 4 | Dxd-a prepared from exatecan | 98.00% | 0.54% |
| | Dxd-b prepared from exatecan | 98.01% | 0.61% |
| Route 5 | Dxd-a' prepared from exitecan | 98.31% | 0.49% |
| | Dxd-b' prepared from exatecan | 98.42% | 0.46% |
| Route of the present disclosure | Example 1, exatecan mesylate | 99.66% | 0.08% |
| | Example 2, exatecan mesylate | 95.46% | 1.07% |

In the prior art, synthesis routes 3, 4, and 5 all use the same intermediate as the present disclosure (compound **5** has the same structure as the compound of formula **VII**), and the final product **LE14** can be prepared from the same intermediate. The final product **LE14** is prepared by the three routes, and the overall yield of the routes and the purity of the final product are summarized in Table 5.

**Table 5. Comparison of Final Product Purity and Overall Yield Using Different Process Routes with Same Compound 5 (VII) as Starting Material**

| Patent Name | Step | Product Purity | Overall Yield Calculated Using **5** or **VII** as Starting Material |
|---|---|---|---|
| WO2022204947A1 (Route 3) | 5→1 | 97.71% | 10.7% |
| CN 115215921 A (Route 4) | 5→1a→1 | 98.00% | 21.3% |
| | 5→1b→1 | 98.01% | 37.5% |
| CN115385926A (Route 5) | 5→1a→1 | 98.31% | 23.4% |
| | 5→1b→1 | 98.42% | 41.2% |
| Route of the present disclosure | VII→LE14 | 99.66% | 18.1% |

In the synthetic routes of the prior art CN115215921A and CN115385926A, using exatecan as the starting material, the overall yields of the routes to synthesize the final product 1 are both lower than those of the present disclosure, as shown in Table 6.

**Table 6. Comparison of Overall Yield of Different Process Routes Using Exatecan as Starting Material**

| Patent Name | Step | Product Purity | Overall Yield Using Exatecan as Starting Material |
|---|---|---|---|
| CN 115215921 A (Route 4) | Exatecan→1a→1 | 98.00% | 8.2% |
| | Exatecan→1b→1 | 98.01% | 18.0% |
| CN115385926A (Route 5) | Exatecan→1a→1 | 98.31% | 6.5% |
| | Exatecan→1b→1 | 98.42% | 18.3% |
| Route of the present disclosure | Exatecan→1 | 99.66% | 44.3% |

From Tables 5 and 6, it can be seen that the purity of the products obtained from Route 3, Route 4, and Route 5 is far inferior to that of the present disclosure. Route 3 uses DXd, which is more expensive than exatecan, as the payload source. Although it saves reaction steps, the yield of Route 3 is significantly lower than that of the present disclosure, and the utilization rate of DXd is not high. In Route 4, Route 5, and the synthetic route of the present disclosure, exatecan is a key material with the largest cost share in each process route and is also the main cost control point. Both Route 4 and Route 5 involve the derivatization of exatecan, which not only introduces more process reaction steps and cumbersome production operations but also results in significant losses of exatecan during preparation. When calculating the overall yield of the routes using exatecan as the starting material, the results show that both are far lower than that of the present disclosure. Therefore, in Route 4 and Route 5, the utilization rate of the expensive material exatecan is low, leading to higher production costs and making them unsuitable for large-scale industrial production.

The synthetic route of the present disclosure not only provides high-purity compound **LE14,** but also significantly reduces production costs by improving the utilization rate of exatecan, making it more suitable for large-scale industrial production.

Although specific embodiments of the present disclosure are described above, those skilled in the art will appreciate that these are merely examples and that various changes or modifications may be made to these embodiments without departing from the principles and essence of the present disclosure. Therefore, the scope of protection of the present disclosure is defined by the appended claims.

## Claims

1. A preparation method for a compound of formula **I,** comprising the following step: subjecting a compound of formula **II** and a compound of formula **III** or a mesylate thereof to an amide condensation reaction in the presence of a condensing agent, a base, and a solvent to obtain the compound of formula **I;**

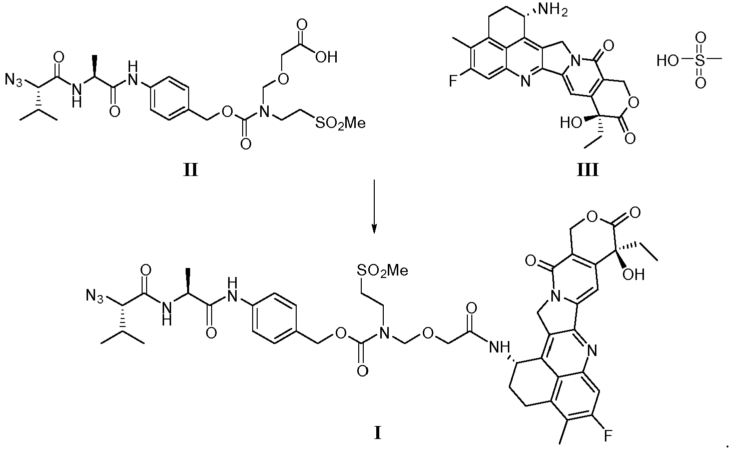

2. The preparation method according to claim 1, wherein the preparation method satisfies one or more of the following conditions:
(1) in the preparation method for the compound of formula **I,** the amide condensation reaction further comprises the following specific steps: dissolving the compound of formula **II** in the solvent, adding the condensing agent, and adding the compound of formula **III** or the mesylate thereof and the base immediately or after reacting for a period of time;
(2) in the preparation method for the compound of formula **I,** the condensation reaction is protected from light throughout the entire process;
(3) in the preparation method for the compound of formula **I,** the molar ratio of the compound of formula **III** to the compound of formula **II** is 0.8 to 1.5, preferably 0.9 to 1.2, and more preferably 0.9 to 1.0;
(4) in the preparation method for the compound of formula **I,** the condensing agent is one or a mixture of any two or more of 4-(4,6-dimethoxy-1,3,5-triazin-2-yl)-4-methylmorpholinium chloride, diethyl cyanophosphonate, diphenylphosphoryl azide, 1-ethyl-(3-dimethylaminopropyl)carbodiimide hydrochloride, 1-hydroxybenzotriazole, 2-(7-azabenzotriazol-1-yl)-*N,N,N',N*'-tetramethyluronium hexafluorophosphate, *O*-(benzotriazol-1-yl)-*N,N,N',N*'-tetramethyluronium hexafluorophosphate, *O*-(6-chlorobenzotriazol-1-yl)-*N,N,N',N*'-tetramethyluronium hexafluorophosphate, 2-(1*H*-benzotriazol-1-yl)-1,1,3,3-tetramethyluronium tetrafluoroborate, 2-succinimido-1,1,3,3-tetramethyluronium tetrafluoroborate, and 2-(5-norborene-2,3-dicarboximido)-1,1,3,3-tetramethyluronium tetrafluoroborate; preferably, the condensing agent is 4-(4,6-dimethoxy-1,3,5-triazin-2-yl)-4-methylmorpholinium chloride;
(5) in the preparation method for the compound of formula **I,** the molar ratio of the condensing agent to the compound of formula **II** is 1 to 3, preferably 1 to 1.5;
(6) in the preparation method for the compound of formula **I,** the base is an organic base, an inorganic base, or a mixture thereof, preferably an organic base; wherein the organic base is preferably one or a mixture of any two or more of *N,N-*diisopropylethylamine, triethylamine, and pyridine, further preferably *N,N-*diisopropylethylamine; the inorganic base is preferably one or a mixture of any two or more of alkali metal hydroxides, alkali metal carbonates, and alkali metal phosphates, further preferably one or a mixture of any two or more of sodium bicarbonate, sodium carbonate, potassium carbonate, cesium carbonate, sodium hydroxide, and potassium hydroxide;
(7) in the preparation method for the compound of formula **I,** the molar ratio of the base to the compound of formula **II** is 1 to 10, preferably 1 to 6, more preferably 1 to 3, and most preferably 1.5;
(8) in the preparation method for the compound of formula **I,** the solvent is one or a mixture of any two or more of *N,N-*dimethylformamide, dimethyl sulfoxide, tetrahydrofuran, and 1,4-dioxane, preferably *N*,*N*-dimethylformamide;
(9) in the preparation method for the compound of formula **I,** the temperature of the condensation reaction is 20°C to 50°C, preferably 20°C to 30°C;
(10) in the preparation method for the compound of formula **I,** the condensation reaction is carried out under the protection of an inert gas, such as in a nitrogen or helium atmosphere;
(11) the preparation method for the compound of formula **I** further comprises the following post-treatment step after the completion of the condensation reaction: concentrating the organic phase to obtain a crude product of the compound of formula **I;** preferably, purifying the crude product of the compound of formula **I** by silica gel column chromatography to obtain a product of the compound of formula **I;** more preferably, the eluent for the silica gel column chromatography is a mixed solvent of chloroform and methanol, wherein the volume ratio of chloroform to methanol is (100 to 10): 1, and further preferably 10: 1.

3. The preparation method according to claim 2, wherein the preparation method for the compound of formula **I** further comprises a preparation method for the compound of formula **II,** and the preparation method for the compound of formula **II** comprises the following step: subjecting a compound of formula **IV** to a deprotection reaction in the presence of a deprotecting agent and in a solvent to obtain the compound of formula **II;** 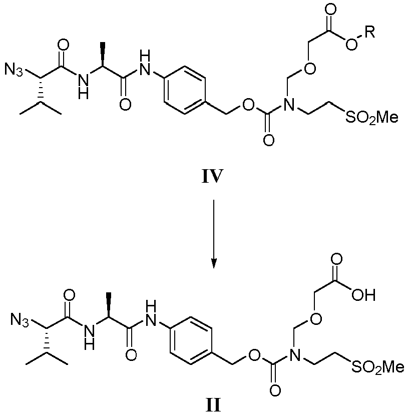
wherein R is C₁-C₆ alkyl substituted by -Si(C₁-C₆)₃;
preferably, the preparation method for the compound of formula **I** further comprises a preparation method for the compound of formula **IV,** and the preparation method for the compound of formula **IV** comprises the following step: subjecting a compound of formula **VI** to an etherification reaction with reagent **V** in a solvent and in the presence of a base to obtain the compound of formula **IV;** 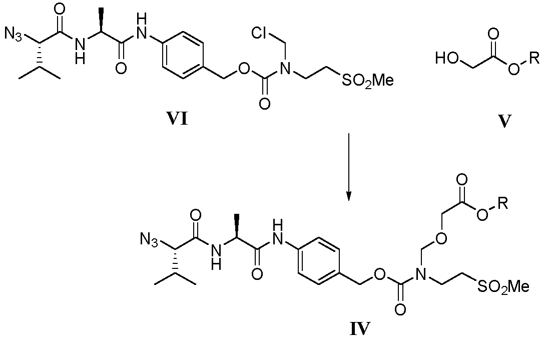
wherein R is C₁-C₆ alkyl substituted by -Si(C₁-C₆)₃;
further preferably, the preparation method for the compound of formula **I** further comprises a preparation method for the compound of formula **VI,** and the preparation method for the compound of formula **VI** comprises the following step: subjecting a compound of formula **VII** to a substitution reaction with paraformaldehyde and trimethylsilyl chloride in a solvent to obtain the compound of formula **VI;**

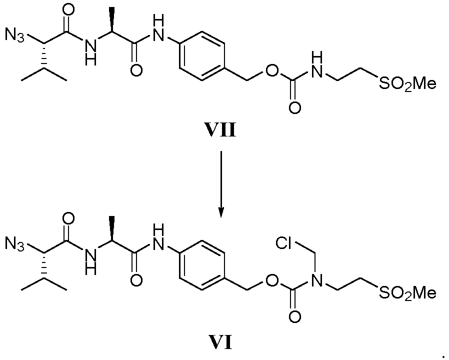

4. The preparation method according to claim 3, wherein the preparation method satisfies one or more of the following conditions:
(1) in the preparation method for the compound of formula **II,** the deprotection reaction further comprises the following specific steps: dissolving the compound of formula **IV** in the solvent, adding the deprotecting agent, and initiating the reaction by heating; or dissolving the deprotecting agent in the solvent, adding the compound of formula **IV,** and initiating the reaction by heating;
(2) in the preparation method for the compound of formula **II,** the C₁-C₆ alkyl substituted by -Si(C₁-C₆)₃ is trimethylsilylethyl or tert-butyldimethylsilylethyl; preferably, the C₁-C₆ alkyl substituted by -Si(C₁-C₆)₃ is trimethylsilylethyl;
(3) in the preparation method for the compound of formula **II,** the deprotecting agent is a fluorinating reagent, and the fluorinating reagent is tetrabutylammonium fluoride, tetramethylammonium fluoride, tetrabutylammonium fluoride/acetic acid, pyridine hydrofluoride complex, *tert*-butylammonium fluoride, *tert*-butylammonium fluoride/acetic acid, tetraethylammonium fluoride, or a commercially available tetramethylammonium fluoride/tetrahydrofuran solution, tetraethylammonium fluoride/tetrahydrofuran solution, tetrabutylammonium fluoride/tetrahydrofuran solution, or one or more of silicon tetrafluoride, potassium fluoride, sodium fluoride, lithium fluoride, and cesium fluoride; preferably, the fluorinating reagent is a 1 M tetrabutylammonium fluoride/tetrahydrofuran solution or potassium fluoride;
(4) in the preparation method for the compound of formula **II,** the molar ratio of the deprotecting agent to the compound of formula **IV** is 1 to 5, preferably 1 to 3, more preferably 1.1 to 2.0, and most preferably 1.5;
(5) in the preparation method for the compound of formula **II,** the solvent is one or a mixture of any two or more of purified water, *N,N*-dimethylformamide, dimethyl sulfoxide, tetrahydrofuran, and 1,4-dioxane, preferably *N,N*-dimethylformamide or tetrahydrofuran, and most preferably *N,N-*dimethylformamide;
(6) in the preparation method for the compound of formula **II,** the temperature of the deprotection reaction is 20°C to 80°C, preferably 30°C to 70°C, and more preferably 40°C to 60°C;
(7) in the preparation method for the compound of formula **II,** the deprotection reaction is carried out under the protection of an inert gas, such as in a nitrogen or helium atmosphere;
(8) the preparation method for the compound of formula **II** further comprises a post-treatment step after the completion of the reaction: concentrating the reaction mixture to obtain a crude product of the compound of formula **II;** preferably, purifying the crude product of the compound of formula **II** by silica gel column chromatography to obtain a product of the compound of formula **II;** more preferably, the eluent for the silica gel column chromatography is a mixed solvent of dichloromethane and methanol, wherein the volume ratio of dichloromethane to methanol is (100 to 1):1, preferably (10 to 1):1;
(9) in the preparation method for the compound of formula **IV,** the etherification reaction further comprises the following specific steps: dissolving the compound of formula **VI** in the solvent, adding the base, and adding reagent **V** immediately or after reacting for a period of time;
(10) in the preparation method for the compound of formula **IV,** the molar ratio of the reagent V to the compound of formula **VI** is 1 to 5, preferably 1 to 3, more preferably 1.1 to 1.6, and most preferably 1.5;
(11) in the preparation method for the compound of formula **IV,** the base is an organic base, an inorganic base, or a mixture thereof, preferably an organic base; wherein the organic base is preferably one or a mixture of any two or more of potassium *tert*-butoxide, triethylamine, 4-dimethylaminopyridine, pyridine, and pempidine, more preferably pempidine; the inorganic base is preferably one or a mixture of any two or more of alkali metal hydroxides, alkali metal carbonates, and alkali metal phosphates;
(12) in the preparation method for the compound of formula **IV,** the molar ratio of the base to the compound of formula **VI** is 1 to 5, preferably 1.2 to 4, and more preferably 1.5 to 3;
(13) in the preparation method for the compound of formula **IV,** the solvent is one or a mixture of any two or more of *N,N-*dimethylformamide, dimethyl sulfoxide, tetrahydrofuran, and 1,4-dioxane, preferably tetrahydrofuran or 1,4-dioxane, and further preferably tetrahydrofuran;
(14) in the preparation method for the compound of formula **IV,** the temperature of the etherification reaction is 0°C to 80°C, preferably 40°C to 70°C, and most preferably 60°C;
(15) in the preparation method for the compound of formula **IV,** the etherification reaction is carried out under the protection of an inert gas, such as in a nitrogen or helium atmosphere;
(16) the preparation method for the compound of formula **IV** further comprises post-treatment steps after the completion of the reaction: extracting the reaction mixture and concentrating the organic phase to obtain a crude product of the compound of formula **IV,** or directly concentrating the reaction mixture to obtain a crude product of the compound of formula **IV**; preferably, purifying the crude product of the compound of formula **IV** by silica gel column chromatography to obtain a product of the compound of formula **IV;** more preferably, the eluent for the silica gel column chromatography is a mixed solution of *n*-heptane and ethyl acetate, wherein the volume ratio of n-heptane to ethyl acetate is (20 to 1): 1, preferably (10 to 1): 1;
(17) in the preparation method for the compound of formula **VI,** the molar ratio of paraformaldehyde to the compound of formula **VII** is 1 to 10, preferably 1 to 5, more preferably 1.3 to 3.0, and most preferably 1.3;
(18) in the preparation method for the compound of formula **VI,** the molar ratio of trimethylsilyl chloride to the compound of formula **VII** is 1 to 5, preferably 2 to 4, more preferably 2 to 3, and most preferably 2.5;
(19) in the preparation method for the compound of formula **VI,** the solvent is one or a mixture of any two or more of *N,N-*dimethylformamide, dimethyl sulfoxide, tetrahydrofuran, and 1,4-dioxane, preferably tetrahydrofuran or 1,4-dioxane, and more preferably 1,4-dioxane;
(20) in the preparation method for the compound of formula **VI,** the temperature of the substitution reaction is -10°C to 50°C, preferably 15°C to 35°C, and more preferably 18°C to 25°C;
(21) in the preparation method for the compound of formula **VI,** the substitution reaction is carried out under the protection of an inert gas, such as in a nitrogen or helium atmosphere;
(22) the preparation method for the compound of formula **VI** further comprises post-treatment steps after the completion of the reaction: subjecting the reaction mixture to solid-liquid separation or omitting solid-liquid separation, followed by concentrating the organic phase to obtain the compound of formula **VI;** preferably, directly subjecting the compound of formula **VI** obtained after concentration to subsequent reaction.

5. A preparation method for a compound of formula **VIII**, comprising the following step: subjecting a compound of formula **I** to a reduction reaction with a reducing agent in the presence of a solvent and an acid buffer to obtain the compound of formula **VIII**; 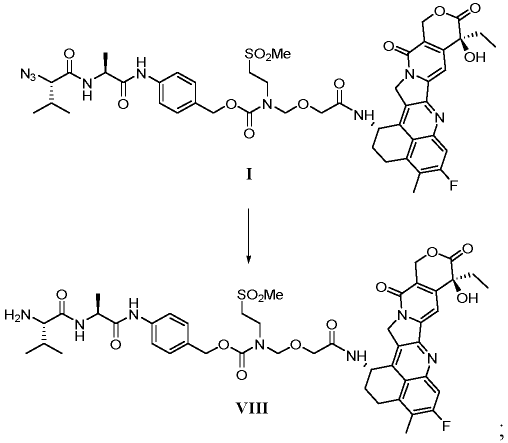 wherein the compound of formula I is prepared by the preparation method according to any one of claims 1 to 4.

6. The preparation method according to claim 5, wherein the preparation method satisfies one or more of the following conditions:
(1) in the preparation method for the compound of formula **VIII,** the reducing agent is triphenylphosphine, tri-*tert*-butylphosphine, or trimethylphosphine, or a triphenylphosphine/tetrahydrofuran solution, a tri-*tert*-butylphosphine/tetrahydrofuran solution, or a trimethylphosphine/tetrahydrofuran solution, preferably a trimethylphosphine/tetrahydrofuran solution, and more preferably a 1 M trimethylphosphine/tetrahydrofuran solution;
(2) in the preparation method for the compound of formula **VIII,** the acid buffer is a sodium acetate buffer or a sodium formate buffer, preferably a sodium acetate buffer; the pH of the acid buffer is preferably 4.0 to 6.0, more preferably 4.5 to 5.5, and even more preferably 5.0;
(3) in the preparation method for the compound of formula **VIII,** the molar ratio of the reducing agent to the compound of formula **I** is 1.0 to 3.0, preferably 1.2 to 1.8, and more preferably 1.5;
(4) in the preparation method for the compound of formula **VIII,** the volume-to-mass ratio of the acid buffer to the compound of formula I is 2 to 10 mL/g, preferably 5 to 10 mL/g, more preferably 7 to 8 mL/g, and most preferably 7.7 mL/g;
(5) in the preparation method for the compound of formula **VIII,** the solvent is an ether solvent, preferably one or a mixture of any two or more of tetrahydrofuran, diethyl ether, 1,4-dioxane, anisole, and methyl *tert*-butyl ether, and further preferably tetrahydrofuran;
(6) in the preparation method for the compound of formula **VIII,** the temperature of the reduction reaction is 0°C to 20°C, preferably 0°C to 10°C;
(7) the preparation method for the compound of formula **VIII** further comprises post-treatment steps after the completion of the reaction: extracting the reaction mixture and concentrating the organic phase to obtain a crude product of the compound of formula **VIII,** or directly concentrating the reaction mixture to obtain a crude product of the compound of formula **VIII**; preferably, purifying the crude product of the compound of formula **VIII** by silica gel column chromatography to obtain a product of the compound of formula **VIII**; more preferably, the eluent for the silica gel column chromatography is a mixed solvent of dichloromethane and methanol, wherein the volume ratio of dichloromethane to methanol is (100 to 1):1, preferably (10 to 1):1.

7. A preparation method for compound **LE14,** comprising the following step: subjecting a compound of formula **VIII** and N-succinimidyl 6-maleimidohexanoate to a coupling reaction in a solvent to obtain the compound **LE14;** 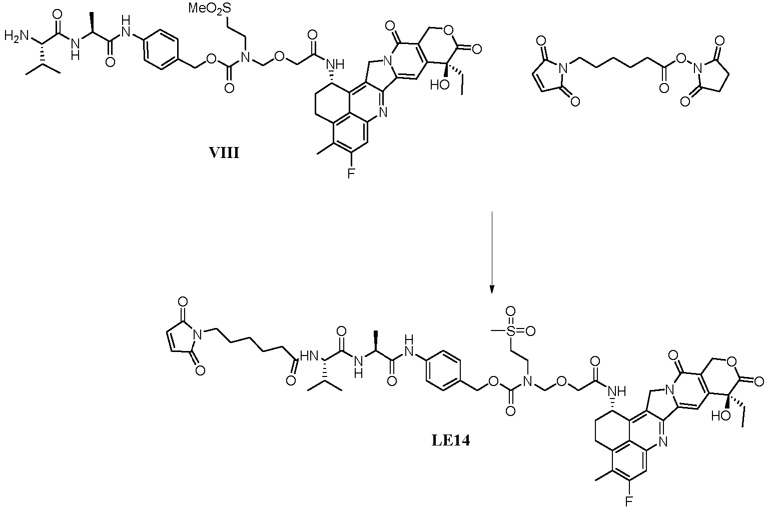 wherein the compound of formula **VIII** is prepared by the preparation method according to claim 5 or 6.

8. The preparation method according to claim 7, wherein the preparation method satisfies one or more of the following conditions:
(1) in the preparation method for the compound **LE14,** the molar ratio of*N*-succinimidyl 6-maleimidohexanoate to the compound of formula **VIII** is 1 to 5, preferably 1 to 3, and more preferably 1.2 to 2.0;
(2) in the preparation method for the compound **LE14,** the solvent is one or a mixture of any two or more of amide solvents, chlorinated alkane solvents, ether solvents, and nitrile solvents, preferably, a chlorinated alkane solvent; wherein the chlorinated alkane solvent is preferably one or a mixture of any two or more of dichloromethane, 1,2-dichloroethane, and chloroform, further preferably dichloromethane;
(3) in the preparation method for the compound **LE14,** the temperature of the coupling reaction is 0°C to 45°C, preferably 25°C to 40°C, and more preferably 30°C to 35°C;
(4) the preparation method for the compound **LE14** further comprises a post-treatment step after the completion of the reaction: concentrating the reaction mixture to obtain a crude product of the compound **LE14;** preferably, purifying the crude product of the compound **LE14** by silica gel column chromatography to obtain a product of the compound **LE14;** more preferably, the eluent for the silica gel column chromatography is a mixed solvent of dichloromethane and methanol, wherein the volume ratio of dichloromethane to methanol is (100 to 1):1, preferably (10 to 1):1.

9. A compound of formula **II**;

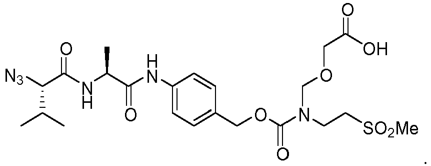

10. A preparation method for a compound of formula **II,** comprising the following step: subjecting a compound of formula **IV** to a deprotection reaction in the presence of a deprotecting agent and in a solvent to obtain the compound of formula **II;** wherein conditions for the reaction are as defined in claim 4; 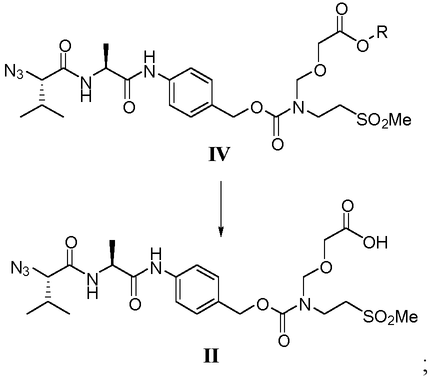 wherein R is as defined in claim 3 or 4.
